# EUROPÄISCHE PATENTSCHRIFT

(11) **EP 2 776 479 B1**
(45) Veröffentlichungstag und Bekanntmachung des Hinweises auf die Patenterteilung: **23.09.2015**
(21) Anmeldenummer: 12781114.9
(22) Anmeldetag: 07.11.2012
(51) Int. Cl.: C08F 220/34, C08F 220/18, C08F 2/32, C09D 7/00, C11D 3/37

(54) **VERDICKER ENTHALTEND MINDESTENS EIN KATIONISCHES POLYMER HERSTELLBAR DURCH INVERSE EMULSIONSPOYLMERISATION**
THICKENER CONTAINING AT LEAST ONE CATIONIC POLYMER WHICH CAN BE OBTAINED BY INVERSE EMULSION POYLMERISATION
ÉPAISSISSANT CONTENANT AU MOINS UN POLYMÈRE CATIONIQUE POUVANT ÊTRE OBTENU PAR POLYMÉRISATION EN ÉMULSION INVERSE

(30) Priorität: 11.11.2011 EP 11188727
(43) Veröffentlichungstag der Anmeldung: 17.09.2014
(73) Patentinhaber: BASF SE, 67056 Ludwigshafen (DE)
(72) Erfinder: LEYRER, Reinhold J., 67125 Dannstadt-Schauernheim (DE); ARISANDY, Christofer, 68549 Ilvesheim (DE); BENLAHMAR, Ouidad, 68163 Mannheim (DE)
(86) Internationale Anmeldenummer: PCT/EP2012/072009
(87) Internationale Veröffentlichungsnummer: WO 2013/068387

(56) Entgegenhaltungen:
- WO-A1-2011/077083
- US-A- 6 107 398

## Beschreibung

Die vorliegende Erfindung betrifft einen Verdicker herstellbar nach einem Verfahren, in dem ein kationisches Polymer durch inverse Emulsionspolymerisation bei einer konstanten Temperatur von mindestens 40 °C hergestellt wird. Bei der inversen Emulsionspolymerisation werden als Komponenten mindestens ein wasserlösliches, ethylenisch ungesättigtes Monomer, umfassend mindestens ein kationisches Monomer, und mindestens ein ethylenisch ungesättigtes Assoziativmonomer eingesetzt. Weiterhin betrifft die vorliegende Erfindung ein Verfahren zur Herstellung des erfindungsgemäßen Verdickers sowie tensidhaltige Formulierungen enthaltend mindestens einen Verdicker. Weitere Erfindungsgegenstände sind die Verwendung der tensidhaltigen Formulierungen, beispielsweise als Weichspüler oder als Flüssigwaschmittel sowie die Verwendung des Verdickers beispielsweise als Viskositätsveränderer.

WO 03/102043 betrifft wässrige Formulierungen enthaltend ein kationisches Polymer, hergestellt aus (i) einem wasserlöslichen, ethylenisch ungesättigten Monomeren oder einer Monomerenmischung umfassend mindestens ein kationisches Monomer, (ii) mindestens einem Quervernetzer in einer Menge von mehr als 50 ppm bezogen auf die Komponente (i), und (iii) mindestens ein Kettentransfermittel. Die wässrigen Formulierungen können als Verdicker in Haushaltsformulierungen eingesetzt werden.

WO 2009/019225 betrifft eine wässrige Dispersion eines alkalilöslichen Copolymers, die sich als Assoziativverdicker eignet. Das Copolymer umfasst einpolymerisierte Einheiten von a) wenigstens einer ethylenisch ungesättigten Carbonsäure, b) wenigstens eines nichtionischen ethylenisch ungesättigten Tensidmonomers, c) wenigstens eines C₁-C₂-Alkylmethacrylats und d) wenigstens eine C₂-C₄-Alkylacrylats, wobei die über die Zahl der Alkylgruppen des Alkylacrylats gemittelte Alkylkettenlänge 2,1 bis 4,0 beträgt. Die Assoiziativverdicker können durch Emulsionspolymerisation hergestellt werden. Die Assoziativverdicker eignen sich zur Verwendung in Wasch- und Reinigungsmitteln.

Liquid Dispersion Polymer (LDP)-Zusammensetzungen werden in WO 2005/097834 offenbart. Diese LDP-Zusammensetzungen umfassen ein hydrophiles, wasserlösliches oder quellbares Polymer mit einem Neutralisierungsgehalt von ungefähr 25 bis ungefähr 100 %, eine nicht-wässrige Trägerphase und ein Öl-in-Wasser-Tensid. Das hydrophile, wasserlösliche oder quellbare Polymer wird vorzugsweise durch Polymerisation, beispielsweise von Acrylsäure oder Methacrylsäure, erhalten. Die LDP-Dispersionen eignen sich zur Herstellung von mikropartikulären Verdickern, wie sie beispielsweise in wässrigen oder organischen Zusammensetzungen, insbesondere in Personal Care oder pharmazeutischen Formulierungen verwendet werden.

WO 2010/078959 betrifft kationische Polymerverdicker bestehend aus einem quervernetzten in Wasser quellbaren kationischen Polymer, enthaltend mindestens ein kationische Monomer und gegebenenfalls nichtionische oder anionische Monomere, wobei das Polymer weniger als 25 % an wasserlöslichen Polymerketten, bezogen auf das Gesamtgewicht des Polymers, umfasst. Weiterhin enthält das Polymer einen Quervernetzer in einer Konzentration von 500 bis 5000 ppm relativ zum Polymer. Das kationische Polymer wird durch inverse Emulsionspolymerisation hergestellt.

WO 2010/079100 offenbart Weichspülerzusammensetzungen enthaltend Polymere gemäß WO 2010/078959.

US 2008/0312343 betrifft inverse Latex-Zusammensetzungen sowie deren Verwendung als Verdicker und/oder Emulgator, beispielsweise zur Herstellung von kosmetischen oder pharmazeutischen Formulierungen. Die inversen Latex-Zusammensetzungen umfassen mindestens 50 bis 80 Gew.-% von mindestens einem linearen, verzweigten oder quervernetzten organischen Polymer (P), mindestens 5 bis 10 Gew.-% eines Emulgatorsystems der Wasser-in-Öl-Art, 5 bis 45 Gew.-% von mindestens einem Öl und bis zu 5 % Wasser. Das Polymer P umfasst neutrale Monomere sowie gegebenenfalls kationische oder anionische Monomere. Die inverse Latex-Zusammensetzung kann gegebenenfalls bis zu 5 Gew.-% eines Emulgatorsystems der Öl-in-Wasser-Art umfassen. Die inversen Latex-Zusammensetzungen können durch inverse Emulsionspolymerisation hergestellt werden.

EP-A 172 025 betrifft eine Dispersion in einer kontinuierlichen flüssigen Phase eines Polymers, das gebildet wird durch Polymerisation von einem ethylenisch ungesättigten Monomeren enthaltend eine hydrophobe Gruppe von mindestens 8 Kohlenstoffatomen und einem damit copolymierisierbaren ethylenisch ungesättigten Monomeren. Die Dispersion ist stabil, im Wesentlichen wasserfrei und enthält mindestens 40 Gew.-% an Polymer. Bei der Polymerisierung können als copolymerisierbares, ethylenisch ungesättigtes Monomer beispielsweise anionische Monomere eingesetzt werden. Die Polymerisation kann als inverse Emulsionspolymerisation durchgeführt werden.

EP-A 172 724 betrifft Polymere, die durch Copolymerisation von a) einem ethylenisch ungesättigten Monomer enthaltend eine hydrophobe Gruppe mit mindestens 8 Kohlenstoffatomen und b) wasserlöslichen ethylenisch ungesättigten Monomeren hergestellt werden. Alle Monomere sind als Gemisch löslich in Wasser und das Polymer wird durch inverse Emulsionspolymerisation hergestellt. Die Polymerpartikel haben eine Trockengröße von < 4 µm. Als Monomerkomponente b) können anionische Monomere wie Acrylsäure in Form der freien Säure oder als wasserlösliches Salz sowie nichtionische Monomere wie Acrylamid verwendet werden.

EP-A 172 723 betrifft ein Verfahren zur Ausflockung einer Suspension unter Verwendung eines wasserlöslichen, im Wesentlichen linearen Polymers mit einer "Ein-Punktintrinsischen Viskosität" von > 3. Das Polymer ist ein Copolymer von zwei oder mehr ethylenisch ungesättigten Monomeren umfassend mindestens 0,5 Gew.-% eines Monomers, das hydrophobe Gruppen enthält. Das Polymer kann auch ein kationisches Polymer sein.

Die der vorliegenden Erfindung zugrundeliegende Aufgabe besteht in der Bereitstellung von neuen Verdickern. Gelöst wird die Aufgabe durch die erfindungsgemäßen Verdicker herstellbar nach einem Verfahren, dadurch gekennzeichnet, dass ein kationisches Polymer durch eine inverse Emulsionspolymerisation von
a) mindestens einem wasserlöslichen ethylenisch ungesättigten Monomeren umfassend mindestens ein kationisches Monomer, gegebenenfalls mindestens ein anionisches Monomer und/oder gegebenenfalls mindestens ein nicht-ionisches Monomer,
b) mindestens einem ethylenisch ungesättigten Assoziativmonomeren,
c) gegebenenfalls mindestens einem Vernetzer,
d) gegebenenfalls mindestens einem Kettentransfermittel,
erhalten wird, wobei die Temperatur während der inversen Emulsionspolymerisation konstant gehalten wird und mindestens 40 °C, vorzugsweise 50 bis 90 °C, beträgt und nach Beendigung der inversen Emulsionspolymerisation die Aktivatorzugabe unter Erhalt des Verdickers erfolgt.

Die erfindungsgemäßen Verdicker zeichnen sich dadurch aus, dass sie über vorteilhafte Eigenschaften hinsichtlich des Depositionings, der Scherverdünnung, der Stabilisierung und/oder der Viskosität (Verdickung) verfügen. Unter Depositioning (Depositionierung) wird die Ablagerung der Wirkstoffe von beispielsweise einem Weichspüler auf einer Faser während eines Waschvorgangs verstanden. Auf die vorliegende Erfindung übertragen bedeutet dies, dass beispielsweise ein erfindungsgemäßer Verdicker, enthaltend mindestens ein kationisches Polymer (Wirkstoff), in einem Weichspüler enthalten ist und der Weichspüler während oder nach dem Waschvorgang eingesetzt wird. Die erfindungsgemäßen Verdicker fördern diese Ablagerung des Wirkstoffes während oder nach dem Waschvorgang in beträchtlichem Ausmaß. Besonders gute Eigenschaften bezüglich des Depositionings können dann erzielt werden, wenn kationische Polymere eingesetzt werden, die auf mindestens einem Assoziativmonomeren, einem kationischen Monomeren und einem nichtionischen Monomeren wie Acrylamid basieren.

Bei der Beurteilung der Scherverdünnung ist es bedeutsam, dass der Verdicker bzw. der entsprechende Weichspüler in seinem Grundzustand sämig und dick ist, während er hingegen beim Rühren dünn ist. Die verbesserte Scherverdünnung wirkt sich positiv auf die Lebensdauer und Eigenschaften von Pumpen bei der Herstellung des Weichspülers aus, fördert die komfortable Dosierung beim Kunden und fördert den rückstandsfreien Einsatz des Weichspülers, insbesondere in den Waschmaschinen, die über eine automatische Dosierungseinrichtung verfügen. Die erfindungsgemäßen Verdicker erhöhen die Stabilität des Verdickers an sich und die der entsprechenden Formulierung. Das Absetzen oder Aufrahmen von Teilchen wird effektiv verhindert, unabhängig davon, ob diese sich in der Größenordnung von Nanometern, Mikrometern oder Millimetern bewegen. Hierzu trägt die vorteilhafte Fließgrenze der erfindungsgemäßen Verdicker bei. Außerdem haben sie den Vorteil, dass die eventuell erforderliche Redispergierung und die Verdickung sehr schnell erreicht wird.

Erfindungsgemäße Verdicker, in denen ein Gemisch von mindestens zwei Aktivatoren enthalten sind, wobei mindestens ein Aktivator einen hohen HLB-Wert und mindestens ein Aktivator einen niedrigen HLB-Wert aufweisen, sind mit einem zusätzlichen Vorteil verbunden. Die Kombination eines solchen Aktivator-Gemisches mit kationischen Polymeren, enthaltend mindestens einen ethylenisch ungesättigten Assoziativmonomeren-Baustein, führt zu spontanen Phaseninversionen (innerhalb von Sekunden) beim Verdünnen eines Verdickers mit Wasser, ohne dass zusätzlicher Energieeintrag, beispielsweise in Form von Rühren, erforderlich ist.

Weiterhin ist es bei erfindungsgemäßen Verdickern von Vorteil, dass das Verhältnis von Assoziativmonomer zum Gesamtpolymer relativ gering ist. Bei Verwendung des Verdickers in tensidhaltigen Formulierungen ist die Wirkung der Assoziativmonomeren bereits bei Mengen von ca. 0,5 % Gew.-% (bezogen auf das Polymer) optimal.

Ein weiterer Vorteil ist darin zu sehen, dass das kationische Polymer des erfindungsgemäßen Verdickers durch inverse Emulsionspolymerisation hergestellt wird, bei der die Temperatur bei mindestens 40 °C konstant gehalten wird, wodurch eine gute Gleichverteilung der Assoziativmonomeren-Bausteine im kationischen Polymer beobachtet wird. Dies ist insbesondere bei geringen Einsatzmengen von beispielsweise 0,1 bis 1 Gew.-% an Assoziativmonomeren vorteilhaft bezüglich der gesamten oben genannten rheologischen Eigenschaften wie Verdickung, Scherverdünnung, Stabilisierung sowie Wasch- und Spüleffekte.

Ausführungsformen der vorliegenden Erfindung, in denen die im Verdicker enthaltenen kationischen Polymere unter Verwendung von wenig oder gar keinem Vernetzer hergestellt werden, sind ebenfalls mit Vorteilen verbunden. Aufgrund der höheren (in Wasser) löslichen Anteile des Polymers ist die Wiederanschmutzung während eines Waschvorgangs verringert. Folglich hat der zu waschende Gegenstand auch nach mehrmaligen Waschprozessen reine Fasern, die effektiv von Schmutzpartikeln befreit sind, so dass kein Vergrauen festgestellt wird. Es ist kein oder nur sehr geringes Anhaften bzw. Umverteilen von Schmutzteilen/Polymeren auf den gewaschenen Gegenständen zu beobachten.

Ein weiterer Vorteil der erfindungsgemäßen Verdicker zeigt sich in tensidhaltigen Formulierungen, insbesondere in tensidhaltigen sauren Formulierungen, weil in diesen Formulierungen eine hohe Verdickungsleistung und/oder eine ausgeprägte Scherverdünnung bereits bei niedrigen Verdickerkonzentrationen (< 1 Gew.-%) erreicht werden.

Im Rahmen der vorliegenden Erfindung bedeuten die Definitionen wie C₁-C₃₀-Alkyl, wie beispielsweise nachstehend für den Rest R₄ in Formel (II) definiert, dass dieser Substituent (Rest) ein Alkylrest mit einer Kohlenstoffatomanzahl von 1 bis 30 ist. Der Alkylrest kann sowohl linear als auch verzweigt sowie gegebenenfalls zyklisch sein. Alkylreste, die sowohl eine zyklische als auch eine lineare Komponente aufweisen, fallen ebenfalls unter diese Definition. Gleiches gilt auch für andere Alkylreste, wie beispielsweise ein C₁-C₄-Alkylrest oder ein C₁₆-C₂₂-Alkylrest. Gegebenenfalls können die Alkylreste auch mit funktionellen Gruppen wie Amino, quarternärem Ammonium, Hydroxy, Halogen, Aryl oder Heteroaryl einfach oder mehrfach substituiert sein. Soweit nicht anders angegebenen, weisen die Alkylreste vorzugsweise keine funktionellen Gruppen als Substituenten auf. Beispiele für Alkylreste sind Methyl, Ethyl, n-Propyl, iso-Propyl, n-Butyl, iso-Butyl, 2-Ethylhexyl, tertiär-Butyl (tert-Bu/t-Bu), Cyklohexyl, Octyl , Stearyl oder Behenyl.

Nachfolgend wird die vorliegende Erfindung weiter präzisiert.

Zunächst werden die Monomerkomponenten näher definiert, die zur Herstellung des im erfindungsgemäßen Verdicker enthaltenen kationischen Polymers verwendet werden. Das inverse Emulsionspolymerisationsverfahren als solches zur Herstellung des kationischen Polymers bzw. des erfindungsgemäßen Verdickers enthaltend mindestens ein kationisches Polymer sowie gegebenenfalls bei der inversen Emulsionspolymerisation bzw. dem Verdickerherstellungsverfahren verwendete Zusatz- oder Hilfsstoffe werden weiter unten im Text näher definiert.

Der erfindungsgemäße Verdicker enthält mindestens ein kationisches Polymer, das durch inverse Emulsionspolymerisation der nachfolgenden Komponenten a) und b) sowie gegebenenfalls c) und d) erhalten wird.

Als Komponente a) wird mindestens ein wasserlösliches, ethylenisch ungesättigtes Monomer, umfassend mindestens ein kationisches Monomer, gegebenenfalls mindestens ein anionisches Monomer und/oder gegebenenfalls mindestens ein nicht-ionisches Monomer verwendet. Kationische Monomere als solche, gegebenenfalls vorhandene anionische Monomere als solche sowie gegebenenfalls vorhandene nicht-ionische Monomere als solche sind dem Fachmann bekannt.

Vorzugsweise ist das kationische Monomer gemäß Komponente a) ausgewählt aus einer Verbindung gemäß Formel (II) 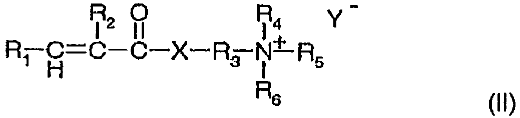 wobei
R₁ gleich H oder C₁ - C₄- Alkyl ist,
R₂ gleich H oder Methyl ist,
R₃ gleich C₁ - C₄ - Alkylen ist,
R₄, R₅ und R₆ unabhängig voneinander H oder C₁ - C₃₀- Alkyl sind,
X gleich -O- oder -NH- ist und
Y gleich Cl; Br; I; Hydrogensulphat oder Methosulfat ist.

Besonders bevorzugte kationische Monomere sind 2-Trimethylammoniumethylacrylatchlorid (TMAEC) oder 2-Trimethylammoniumethylmethacrylatchlorid (TMAEMC). TMAEC wird auch als quarternisiertes Dimethylaminoethylacrylat (DMAEA, MeClq) und TMAEMC als quarternisiertes Dimethylaminoethylmethacrylat (DMAEMA, MeClq) bezeichnet.

In einer Ausführungsform der vorliegenden Erfindung ist es bevorzugt, dass im kationischen Monomer gemäß Formel (II)
i) R₁ und R₂ gleich H oder
ii) R₁ gleich H und R₂ gleich CH₃ sind.

Das in der Komponente a) gegebenenfalls enthaltene anionische Monomer ist vorzugsweise ausgewählt aus Acrylsäure, Methacrylsäure, Itaconsäure, Maleinsäure oder einem Salz davon, insbesondere ist das anionische Monomer Na-Acrylat. Sofern die Komponente a) mindestens ein anionisches Monomer umfasst, ist dieses (bezogen auf die Komponente a)) vorzugsweise zu 0,5 bis 20 Gew.-% enthalten.

Gegebenenfalls kann die Komponente a) mindestens ein nicht-ionisches Monomer umfassen. Außer den nachstehend beschriebenen stickstoffhaltigen Monomeren, wie beispielsweise die Verbindungen gemäß Formel (III), eignen sich auch Ester der vorstehend beschriebenen anionischen Monomere als nicht-ionische Monomere. Solche nicht-ionischen Monomere sind vorzugsweise die Methyl- oder Ethylester der Acryl- oder Methacrylsäure wie Ethylacrylat oder Methylacrylat. Weiterhin bevorzugt sind die entsprechenden mit Dimethylamino-substituierten Ester wie Dimethylaminoethyl(meth)-acrylat.

Vorzugsweise ist im kationischen Polymer das nicht-ionische Monomer gemäß Komponente a) ausgewählt aus N-Vinylpyrrolidon, N-Vinylimidazol oder einer Verbindung gemäß der Formel (III) 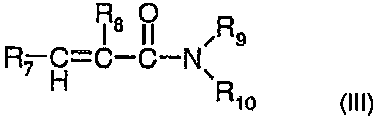 wobei
R₇ gleich H oder C₁ - C₄ - Alkyl ist,
R₈ gleich H oder Methyl ist, und
R₉ und R₁₀ unabhängig voneinander H oder C₁ - C₃₀- Alkyl sind.

Besonders bevorzugt ist das nicht-ionische Monomer Acrylamid, Methacrylamid oder Dialkylaminoacrylamid. Sofern die Komponente a) mindestens ein nicht-ionisches Monomer umfasst, ist dieses vorzugsweise zu 0,5 bis 70 Gew.-% enthalten.

In einer bevorzugten Ausführungsform der vorliegenden Erfindung umfasst im kationischen Polymer die Komponente a) 30 bis 99,5 Gew.-% von mindestens einem kationischen Monomeren und 0,5 bis 70 Gew.-% von mindestens einem nicht-ionischen Monomeren. In einer weiteren bevorzugten Ausführungsform der vorliegenden Erfindung umfasst die Komponente a) 100 Gew.-% von mindestens einem kationischen Monomeren.

Als Komponente b) wird zur Herstellung des kationischen Polymers bei der inversen Emulsionspolymerisation mindestens ein ethylenisch ungesättigtes Assoziativmonomer eingesetzt. Assoziativmonomere als solches sind dem Fachmann bekannt. Geeignete Assoziativmonomere sind beispielsweise in WO 2009/019225 beschrieben. Assoziativmonomere werden auch als Tensidmonomere bezeichnet.

Vorzugsweise ist im kationischen Polymer das ethylenisch ungesättigte Assoziativmonomer gemäß Komponente b) ausgewählt aus einer Verbindung gemäß Formel (I)

R-O-(CH₂-CHR'-O)ₙ-CO-CR"=CH₂ (I)

wobei
R gleich C₈ - C₅₀ - Alkyl, vorzugsweise C₈ - C₃₀ - Alkyl, insbesondere C₁₆ - C₂₂- Alkyl, ist,
R' gleich H oder C₁ - C₄ - Alkyl, vorzugsweise H, ist,
R" gleich H oder Methyl ist,
n eine ganze Zahl von 0 bis 100, vorzugsweise 3 bis 50, insbesondere 25, ist.

Besonders bevorzugt wird als Komponente b) eine Verbindung gemäß Formel (I) eingesetzt, in der
R gleich C₁₆-C₂₂-Alkyl,
R' gleich H,
R" gleich H oder Methyl und
n gleich 25 sind.

Verbindungen gemäß der Formel (I) sind in Lösung kommerziell erhältlich, beispielsweise unter der Bezeichnung Plex 6954 O der Firma Evonik Röhm GmbH. Es handelt sich dabei um Methacrylate von Fettalkoholethoxylaten. Ein geeignetes Fettalkoholethoxylat ist beispielsweise das kommerziell erhältliche Lutensol^{®} AT 25 (BASF SE, Ludwigshafen, Deutschland).

Der Rest R kann in den Verbindungen gemäß Formel (I) auch als Gemisch von Resten mit unterschiedlicher Kettenlänge wie C₁₆ und C₁₈ vorliegen. Ein Beispiel hierfür ist C₁₆-C₁₈-Fettalkohol-(Ethylenglykol)₂₅-ethermethacrylat, wo sowohl C₁₆- als auch C₁₈-Fettalkoholreste (in nicht vernachlässigbaren Mengen) als Gemisch vorliegen. Im Gegensatz dazu liegt beispielsweise in den Verbindungen (gemäß Formel (I)) Behenyl-25-Methacrylat und Cetyl-25-Methacrylat der jeweilige Rest R nicht als Gemisch, sondern als C₂₂- bzw. C₁₆-Kette vor. Andere Kettenlängen kommen nur in Form von Verunreinigungen vor. Die Zahl "25" steht in diesen Verbindungen gemäß Formel (I) für die Größe der Variablen n.

Bei der Herstellung des kationischen Polymers durch inverse Emulsionspolymerisation kann gegebenenfalls als Komponente c) mindestens ein Vernetzer enthalten sein. Geeignete Vernetzer sind dem Fachmann bekannt. Vorzugsweise ist im kationischen Polymer der Vernetzer gemäß Komponente c) ausgewählt aus Divinylbenzol; Tetraallyl-Ammoniumchlorid; Allylacrylaten; Allylmethacrylaten; Diacrylaten und Dimethacrylate von Glykolen oder Polyglykolen; Butadien; 1,7-Octadien, Allyl-Acrylamiden oder Allyl-Methacrylamiden; Bisacrylamidoessigsäure; N,N'-Methylen-bisacrylamid oder Polyolpolyallylethern wie Polyallylsaccharose oder Pentaerythritoltriallylether. Weiterhin geeignet als bevorzugter Vernetzer ist Dialkyldimethylammoniumchlorid.

Weiterhin kann bei der Herstellung des kationischen Polymers durch inverse Emulsionspolymerisation als Komponente d) mindestens ein Kettentransfermittel verwendet werden. Geeignete Kettentransfermittel sind dem Fachmann bekannt. Bevorzugte Kettentransfermittel gemäß Komponente d) sind ausgewählt aus Mercaptan, Milchsäure, Ameisensäure, Isopropanol oder Hypophosphiten.

Vorzugsweise ist im erfindungsgemäßen Verdicker mindestens ein kationisches Polymer enthalten, das herstellbar ist durch inverse Emulsionspolymerisation von.
a) 20 bis 99,99 Gew.-%, bevorzugt 90 bis 99,95 Gew.-% (bezogen auf das Polymer), mindestens eines wasserlöslichen ethylenisch ungesättigten Monomeren umfassend mindestens ein kationisches Monomer, gegebenenfalls mindestens ein anionisches Monomer und/oder gegebenenfalls mindestens ein nicht-ionisches Monomer,
b) 0,01 bis 80 Gew.-%, bevorzugt 0,05 bis 5 Gew.-%, besonders bevorzugt 0,1 bis 1 Gew.-% (bezogen auf das Polymer) mindestens eines ethylenisch ungesättigten Assoziativmonomeren,
c) 0 bis 0,3 Gew.-%, bevorzugt 0,01 bis 0,1 Gew.-% (bezogen auf das Polymer) gegebenenfalls mindestens eines Vernetzers,
d) 0 bis 0,3 Gew.-%, bevorzugt 0,01 bis 0,1 Gew.-% (bezogen auf das Polymer) gegebenenfalls mindestens eines Kettentransfermittels.

In einer weiteren Ausführungsform der vorliegenden Erfindung betragen die in Wasser löslichen Anteile des kationischen Polymers mehr als 25 Gew.-% (bezogen auf das Gesamtgewicht des kationischen Polymers), insbesondere dann, wenn wenig oder gar kein Vernetzer zusätzlich zu dem Assoziativmonomer verwendet wird. Vorzugsweise sind mehr als 40 Gew.-%, insbesondere 70 bis 100 Gew.-%, des kationischen Polymers in Wasser löslich. Die Löslichkeit des kationischen Polymers wird nach dem Fachmann bekannten Methoden bestimmt, wobei das im erfindungsgemäßen Verdicker enthaltende kationische Polymer mit einer definierten Menge an Wasser versetzt wird (siehe beispielsweise EP-A 343 840 oder vorzugsweise die Bestimmungsmethode des Sedimentationskoeffizienten in der Dimension Svedberg (sved) gemäß P. Schuck, 'Size-distribution analysis of macromolecules by sedimentation velocity ultracentrifugation and Lamm equation modeling, Biophysical Journal 78,(3) (2000), 1606-1619).

Vorzugsweise beträgt bei dieser Ausführungsform der bei der inversen Emulsionspolymerisation des kationischen Polymers verwendete Anteil an Vernetzer (Komponente c)) < 10 Gew.-% (bezogen auf die Gesamtmenge an Komponenten a) bis d)). Besonders bevorzugt wird bei der inversen Emulsionspolymerisation des kationischen Polymers kein Vernetzer eingesetzt.

Der erfindungsgemäße Verdicker enthält als weitere Komponente mindestens einen Aktivator. Aktivatoren als solche sind dem Fachmann prinzipiell bekannt.

Als Aktivator sind vorzugsweise Tenside geeignet, beispielsweise anionische, nichtionische, kationische und/oder amphotere Tenside, die beispielsweise in WO 2009/019225 offenbart. sind. Vorzugsweise werden anionische und/oder nichtionische Tenside eingesetzt.

Als nichtionische Tenside werden vorzugsweise Fettalkoholalkoxylate eingesetzt. Fettalkoholalkoxylate werden auch als Polyalkylenglykolether bezeichnet. Bevorzugte Fettalkoholalkoxylate sind alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder verzweigt, bevorzugt in 2-Stellung methylverzweigt sein kann bzw. lineare und methyl-verzweigte Reste im Gemisch enthalten kann, so wie sie üblicherweise in Oxoalkoholresten vorliegen. Insbesondere sind jedoch Alkoholethoxylate mit linearen Resten aus Alkoholen nativen oder technischen Ursprungs mit 12 bis 18 C-Atomen, zum Beispiel aus Kokos-, Palm-, Talgfett- oder Oleylalkohol - oder Gemischen davon, wie beispielsweise aus Castoröl ableitbar - und durchschnittlich 2 bis 8 EO pro Mol Alkohol bevorzugt. Zu den bevorzugten ethoxylierten Alkoholen gehören beispielsweise C₁₂-C₁₄-Alkohole mit 3 EO, 4 EO oder 7 EO, C₉-C₁₁-Alkohol mit 7 EO, C₁₃-C₁₅-Alkohole mit 3 EO, 5 EO, 7 EO oder 8 EO, C₁₂-C₁₈-Alkohole mit 3 EO, 5 EO oder 7 EO und Mischungen aus diesen, wie Mischungen aus C₁₂-C₁₄-Alkohol mit 3 EO und C₁₂-C₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte nichtionische Tenside ("Niotenside") einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside oder Alkylpolyglykoside eingesetzt werden. Unter Alkylglykoside bzw. Alkylpolyglykoside versteht der Fachmann generell Verbindungen, die sich aus mindestens einem Alkylfragment und mindestens einem Zucker- oder Polyzuckerfragment zusammensetzen. Die Alkylfragmente leiten sich vorzugsweise von Fettalkoholen mit einer Kohlenstoffatomzahl von 12 bis 22 und die Zuckerfragmente vorzugsweise von Glucose, Sucrose oder Sorbitan ab.

Beispielsweise können Alkylglykoside der allgemeinen Formel (1)

R¹O(G)ₓ (1)

eingesetzt werden, worin R¹ für einen primären geradkettigen oder methylverzweigten, insbesondere in 2-Stellung methylverzweigten aliphatischen Rest mit 8 bis 22, vorzugsweise 12 bis 18 C-Atomen steht und G für eine Glykosideinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (2), 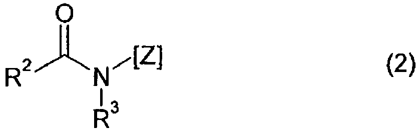 worin R²C(=O) für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (3) 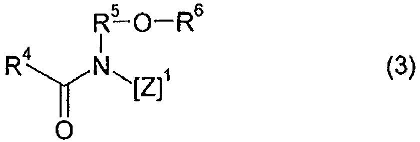 worin R⁴ für einen linearen oder verzweigten Alkyl- oder Alkenylrest mit 7 bis 12 Kohlenstoffatomen, R⁵ für einen linearen, verzweigten oder cyclischen Alkylenrest mit 2 bis 8 Kohlenstoffatomen oder einen Arylenrest mit 6 bis 8 Kohlenstoffatomen und R⁶ für einen linearen, verzweigten oder cyclischen Alkylrest oder einen Arylrest oder einen Oxy-Alkylrest mit 1 bis 8 Kohlenstoffatomen steht, wobei C₁-C₄-Alkyl- oder Phenylreste bevorzugt sind, und [Z]¹ für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z]¹ wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielweise gemäß WO-A-95/07331 durch Umsetzung mit Fettsäuremethylestern in Gegenwart eines Alkoxids als Katalysator in die gewünschten Polyhydroxyfettsäureamide überführt werden.

Als anionische Tenside ("Aniontenside") werden beispielsweise solche vom Typ der Sulfonate und Sulfate eingesetzt. Als Tenside vom Sulfonat-Typ kommen dabei Alkylbenzolsulfonate, vorzugsweise C₉-C₁₃-Alkylbenzolsulfonate, Olefinsulfonate, d.h. Gemische aus Alken- und Hydroxyalkansulfonaten sowie Disulfonaten, wie man sie beispielsweise aus C₁₂-C₁₈-Monooiefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, vorzugsweise sekundäre Alkansulfonate, die beispielsweise aus C₁₂-C₁₈-Alkanen durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Weitere geeignete Aniontenside sind Fettalkoholsulfate, beispielsweise Alk(en)ylsulfate. Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₈-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇-C₂₁-Alkohole, wie 2-Methyl-verzweigte C₉-C₁₁-Alkohole mit im Durchschnitt 3,5 Mol Ethylenoxid (EO) oder C₁₂-C₁₈-Fettalkohole mit 1 bis 4 EO, sind geeignet.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈-C₁₈- Fettalkoholreste oder Mischungen aus diesen. Insbesondere bevorzugte Sulfosuccinate enthalten einen Fettalkoholrest, der sich von ethoxylierten Fettalkoholen ableitet. Dabei sind wiederum Sulfosuccinate, deren Fettalkohol-Reste sich von ethoxylierten Fettalkoholen mit engerr Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Weitere geeignete Aniontenside sind Alkylcarboxylaten, beispielsweise die Natriumsalze von gesättigten oder ungesättigten Fettsäuren, wobei der Alkylrest des Alkylcarboxylats vorzugsweise linear ist.

Im Rahmen der vorliegenden Erfindung ist der Aktivator vorzugsweise ausgewählt aus Fettalkoholalkoxylaten, Alkylglykosiden, Alkylcarboxylaten, Alkylbenzolsulfonaten, sekundären Alkansulfonaten und Fettalkoholsulfaten, besonders bevorzugt ausgewählt aus Fettalkoholalkoxylaten. Ein Beispiel für ein bevorzugtes Fettalkoholalkoxylat ist C₆-C₁₇(sekundär)-Poly(3-6)ethoxylat.

Weiterhin ist es im Rahmen der vorliegenden Erfindung bevorzugt, einen Aktivator einzusetzen, der einen (relativ) hohen HLB-Wert (Hydrophil-Lipophil-Balance-Wert) aufweist. Vorzugsweise hat der Aktivator einen HLB-Wert von 7 bis 18, mehr bevorzugt von 8 bis 15 und besonders bevorzugt von 9 bis 13.

Aktivatoren mit einem hohen HLB-Wert sind vorzugsweise i) Fettalkoholalkoxylate aus sekundären Alkoholen oder Gemischen von Alkoholen mit 12 bis 18 C-Atomen und Ethylenoxid oder Propylenoxid sowie ii) Alkylglykoside aus Sucrose und C₈ bis C₂₂-Fettalkoholen. Beispiele für solche Aktivatoren sind die kommerziell erhältlichen Synperonic 87K der Croda GmbH, Herrenpfad-Süd 33, 41334 Nettetal, Germany; Croduret 40 oder andere ethoxylierte hydrogenierte Castoröle (Rizinusöle) wie Etocas 40 oder Crodesta F110, alle von Croda.

In einer weiteren Ausführungsform der vorliegenden Erfindung ist es bevorzugt, ein Gemisch von mindestens zwei Aktivatoren einzusetzen, wobei mindestens ein Aktivator einen hohen HLB-Wert und mindestens ein Aktivator einen niedrigen HLB-Wert aufweist. Der Aktivator mit einem hohen HLB-Wert hat vorzugsweise einen HLB-Wert von > 12 bis 20 und der Aktivator mit einem niedrigen HLB-Wert hat vorzugsweise einen HLB-Wert von 1 bis 12. In dieser Ausführungsform können der Aktivator mit einem hohen HLB-Wert und der Aktivator mit einem niedrigen HLB-Wert in beliebigen, dem Fachmann bekannten Verhältnissen zueinander vorliegen. Vorzugsweise wird in dem Gemisch 20 bis 50 Gew.-% an Aktivator mit hohem HLB-Wert und 50 bis 80 Gew.-% Aktivator mit niedrigem HLB-Wert eingesetzt. Weiterhin vorzugsweise wird dieses Verhältnis von Aktivator mit hohem HLB-Wert zu Aktivator mit niedrigem HLB-Wert so eingestellt, dass der Gesamt-HLB-Wert 7 bis 18, mehr bevorzugt 8 bis 15 und besonders bevorzugt von 9 bis 13 beträgt.

In diesen Gemischen von mindestens zwei Aktivatoren werden als Aktivatoren mit einem hohen HLB-Wert vorzugsweise Alkylglykoside oder Polyalkylglykoside oder Polyalkyloligoethylenoxidglykosid auf Basis von Sucrose oder Sorbitan und C₈ bis C₂₂-Fettalkoholen wie Polyethyleneglykolsorbitanmonostearat oder Polyoxyethylensorbitanmonostearat eingesetzt. Beispiele für solche Aktivatoren sind die kommerziell erhältlichen Crillet 1, Crillet 3 oder Crodesta F160 , alle von Croda erhältlich.. Als Aktivatoren mit einem niedrigen HLB-Wert werden vorzugsweise Alkylglykoside aus Sucrose oder Sorbitan und C₈ bis C₂₂-Fettalkoholen oder Fettsäuren, wie Sorbitan-Laurat oder Sorbitan-Stearat, eingesetzt. Beispiele für solche Aktivatoren sind die kommerziell erhältlichen Crill 1, Crill 3 oder Crodesta F10 von Croda.

Das Verhältnis von Aktivator zu kationischem Polymer kann im Rahmen der vorliegenden Erfindung in beliebigen, dem Fachmann bekannten Werten eingestellt werden. Bevorzugt beträgt das Verhältnis von Aktivator zum kationischen Polymer > 10 : 100 [Gew.-%/Gew.-%], mehr bevorzugt 10,5 bis 50 : 100 [Gew.-%/Gew.-%], besonders bevorzugt 11,5 bis 20 : 100 [Gew.-%/Gew.-%].

In den erfindungsgemäßen Verdickern können neben dem kationischen Polymer und dem Aktivator noch weitere Komponenten enthalten sein. Geeignete weitere Komponenten werden im nachfolgenden Text im Rahmen der Herstellung des Verdickers bzw. des kationischen Polymers näher definiert. Geeignete weitere Komponenten können beispielsweise Öle und Lösungsmittel sein.

Im erfindungsgemäßen Verdicker kann das kationische Polymer in der Ölphase dispergiert vorliegen, vorzugsweise als inverse Dispersion, Wasser-in-Öl-Dispersion oder als dispergiertes wasserfreies kationisches Polymer in Öl.

Im Rahmen der vorliegenden Erfindung wird das kationische Polymer durch inverse Emulsionspolymerisation hergestellt. Die inverse Emulsionspolymerisation als solche ist dem Fachmann bekannt. Unter inverser Emulsionspolymerisation versteht der Fachmann allgemein Polymerisationsverfahren gemäß der nachfolgenden Definition: Es werden die hydrophilen Monomere in einer hydrophoben Ölphase dispergiert. Die Polymerisation erfolgt direkt in diesen hydrophilen Monomer-Teilchen durch Zugabe von Initiator.

Weiterhin wird im Rahmen der vorliegenden Erfindung die Temperatur während der inversen Emulsionspolymerisation konstant gehalten, wobei die Temperatur mindestens 40 °C, vorzugsweise 50 bis 90 °C beträgt. Normalerweise wird die Temperaturobergrenze von 150 °C bei der inversen Emulsionspolymerisation nicht überschritten.

Sofern im Rahmen der vorliegenden Erfindung bei einer inversen Emulsionspolymerisation, die Temperatur konstant gehalten wird, bedeutet dies, dass ab dem Beginn der inversen Emulsionspolymerisation die Temperatur auf einem konstanten Wert gehalten wird. Schwankungen von +/- 5 °C, vorzugsweise +/- 2 °C und insbesondere +/- 1 °C während des Polymerisationsvorganges werden als konstante Temperatur angesehen (bezogen auf den gewünschten konstanten Temperaturwert). Die Temperatur wird solange konstant gehalten, bis die inverse Emulsionspolymerisation beendet ist, vorzugsweise ist dies der Fall nach einer Umsetzung von mehr als 90 % der eingesetzten Monomere, mehr bevorzugt mehr als 95 Gew.-% und besonders bevorzugt bei Vollumsatz (100 Gew.-%). Die Temperatur kann konstant gehalten werden, indem die entstehende Reaktionswärme durch Kühlung abgeführt wird. Der Start der Polymerisation ist normalerweise die Zugabe des Polymerisationsinitiators, vorzugsweise die Zugabe eines Redoxinitiatorsystems. Normalerweise wird zunächst das System auf die gewünschte Temperatur aufgeheizt und unter Rühren gewartet, bis die Temperatur konstant ist. Anschließend erfolgt die Zugabe des Polymerisationsinitiators, wodurch der Polymerisationsprozess in Gang gesetzt wird. In einer Ausführungsform der vorliegenden Erfindung wird die Temperatur auf einem Wert konstant gehalten, der oberhalb des Schmelzpunktes des eingesetzten Assoziativmonomeren liegt.

Nach Beendigung der inversen Emulsionspolymerisation erfolgt die Aktivatorzugabe zum kationischen Polymer (bzw. zu dem das kationische Polymer enthaltenden Reaktionsgemisch) unter Erhalt des erfindungsgemäßen Verdickers. Der Aktivatorzugabe erfolgt nach dem Fachmann bekannten Schritten, beispielsweise in einer oder mehreren Portionen, wobei gegebenenfalls auch weitere Komponenten zusammen mit dem Aktivator zugegeben werden können.

Zur inversen Emulsionspolymerisation wird ein geeigneter Polymerisationsinitiator verwendet. Redox-Initiatoren und/oder thermisch aktivierbare radikalische Polymerisationsinitiatoren sind bevorzugt.

Geeignete thermisch aktivierbare radikalische Initiatoren bzw die oxidative Komponente des Redox-Initiatorpaares sind vor allem solche des Peroxy- und Azotyps. Hierzu zählen unter anderem Wasserstoffperoxid, Peressigsäure, t-Butylhydroperoxid, Di-t-butylperoxid, Dibenzoylperoxid, Benzoylhydroperoxid, 2,4-Dichlorbenzoylperoxid, 2,5-Dimethyl-2,5-bis(hydroperoxy)hexan, Perbenzoesäure, t-Butylperoxypivalat, t-Butylperacetat, Dilauroylperoxid, Dicapryloylperoxid, Distearoylperoxid, Dibenzoylperoxid, Diisopropylperoxydicarbonat, Didecylperoxydicarbonat, Dieicosylperoxydicarbonat, Di-t-butylperbenzoat, Azobisisobutyronitril, 2,2'-Azobis-2,4-dimethylvaleronitril, Ammoniumpersulfat, Kaliumpersulfat, Natriumpersulfat und Natriumperphosphat.

Die Persulfate (Peroxodisulfate), insbesondere Natriumpersulfat, sind am meisten bevorzugt.

Bei der Durchführung der inversen Emulsionspolymerisation wird der Initiator in ausreichender Menge verwendet, um die Polymerisationsreaktion zu initiieren. Der Initiator wird üblicherweise in einer Menge von etwa 0,01 bis 3 Gew.-%, bezogen auf das Gesamtgewicht der eingesetzten Monomere, verwendet. Die Initiatormenge beträgt vorzugsweise etwa 0,05 bis 2 Gew.-% und insbesondere 0,1 bis 1 Gew.-%, bezogen auf das Gesamtgewicht der eingesetzten Monomere.

Die inverse Emulsionspolymerisation kann sowohl als Batch-Prozess als auch in Form eines Zulaufverfahrens durchgeführt werden. Bei der Zulauffahrweise kann man zumindest einen Teil des Polymerisationsinitiators vorlegen, erhitzt auf Polymerisationstemperatur und führt anschließend den Rest des Polymerisationsansatzes, üblicherweise über mehrere getrennte Zuläufe, von denen einer oder mehrere die Monomeren in reiner oder emulgierter Form enthalten, kontinuierlich oder stufenweise unter Aufrechterhaltung der Polymerisation zu. Vorzugsweise erfolgt der Monomerzulauf in Form einer inversen Monomeremulsion. Parallel zum Monomerzulauf kann weiterer Polymerisationsinitiator zudosiert werden.

In bevorzugten Ausführungsformen legt man die gesamte Initatormenge vor, d. h. parallel zum Monomerzulauf erfolgt keine weitere Initiatordosierung.

In einer bevorzugten Ausführungsform legt man daher den thermisch aktivierbaren radikalischen Polymerisationsinitiator vollständig vor und lässt das Monomerengemisch, vorzugsweise in Form einer inversen Monomeremulsion, zulaufen. Bevor man den Zulauf des Monomerengemisches startet, bringt man die Vorlage auf die Aktivierungstemperatur des thermisch aktivierbaren radikalischen Polymerisationsinitiators oder eine höhere Temperatur, mindestens jedoch auf 40 °C, und hält die entsprechende Temperatur konstant. Als Aktivierungstemperatur wird die Temperatur angesehen, bei der nach einer Stunde mindestens die Hälfte des Initiators zerfallen ist.

Gemäß einer anderen bevorzugten Herstellungsart erhält man das kationische Polymer durch inverse Emulsionspolymerisation eines Monomerengemisches in Gegenwart eines Redoxinitiatorsystems. Ein Redoxinitiatorsystem umfasst wenigstens eine Oxidationsmittelkomponente und wenigstens eine Reduktionsmittelkomponente, wobei im Reaktionsmedium vorzugsweise zusätzlich Schwermetallionen als Katalysator vorhanden sind, beispielsweise Cer-, Mangan- oder Eisen(II)-salze.

Geeignete Oxidationsmittelkomponenten sind beispielsweise Peroxide und/oder Hydroperoxide wie Wasserstoffperoxid, tert.-Butylhydroperoxid, Cumolhydroperoxid, Pinanhydroperoxid, Diisopropylphenylhydroperoxid, Dicyclohexylpercarbonat, Dibenzoylperoxid, Dilauroylperoxid und Diacetylperoxid. Wasserstoffperoxid und tert.-Butylhydroperoxid sind bevorzugt.

Geeignete Reduktionsmittelkomponenten sind Alkalimetallsulfite, Alkalimetalldithionite, Alkalimetallhyposulfite, Natriumhydrogensulfit, Rongalit C (Natriumformaldehydsulfoxylat), Mono- und Dihydroxyaceton, Zucker (z. B. Glucose oder Dextrose), Ascorbinsäure und ihre Salze, Acetonbisulfit-Addukt und/oder ein Alkalimetallsalz der Hydroxymethansuifinsäure. Natriumhydrogensulfit oder Natriummetabisulfit sind bevorzugt.

Als Reduktionsmiltelkomponente bzw. Katalysator eignen sich auch Eisen(II)-salze wie z.B. Eisen(II)-sulfat, Zinn(II)-salze wie z.B. Zinn(II)-chlorid, Titan(III)-salze wie Titan(III)-sulfat.

Die Einsatzmengen an Oxidationsmittel betragen 0,001 bis 5,0 Gew.-%, vorzugsweise von 0,005 bis 1,0 Gew.-% und besonders bevorzugt von 0,01 bis 0,5 Gew.-%, bezogen auf das Gesamtgewicht der eingesetzten Monomere. Reduktionsmittel werden in Mengen von 0,001 bis 2,0 Gew.-%, vorzugsweise von 0,005 bis 1,0 Gew.-% und besonders bevorzugt von 0,01 bis 0,5 Gew.-% eingesetzt, bezogen auf das Gesamtgewicht der eingesetzten Monomere.

Ein besonders bevorzugtes Redoxinitiatorsystem ist das System Natriumperoxodisulfat/ Natriumhydrogensulfit, z. B. 0,001 bis 5,0 Gew.-% Natriumperoxodisulfat und 0,001 bis 2,0 Gew.-% Natriumhydrogensulfit, insbesondere 0,005 bis 1,0 Gew.-% Natriumperoxodisulfat und 0,005 bis 1,0 Gew.-% Natriumhydrogensulfit, besonders bevorzugt 0,01 bis 0,5 Gew.-% Natriumperoxodisulfat und 0,01 bis 0,5 Gew.-% Natriumhydrogensulfit.

Ein weiteres besonders bevorzugtes Redoxinitiatorsystem ist das System t-Butylhydroperoxid/Wasserstoffperoxid/Ascorbinsäure, z. B. 0,001 bis 5,0 Gew.-% t-Butylhydroperoxid, 0,001 bis 5,0 Gew.-% Wasserstoffperoxid und 0,001 bis 2,0 Gew.-% Ascorbinsäure, insbesondere 0,005 bis 1,0 Gew.-% t-Butylhydroperoxid, 0,005 bis 1,0 Gew.-% Wasserstoffperoxid und 0,005 bis 1,0 Gew.-% Ascorbinsäure, besonders bevorzugt 0,01 bis 0,5 Gew.-% t-Butylhydroperoxid, 0,01 bis 0,5 Gew.-% Wasserstoffperoxid und 0,01 bis 0,5 Gew.-% Ascorbinsäure.

Vorzugsweise wird das kationische Polymer durch inverse Emulsionspolymerisation hergestellt, indem zunächst eine wässrige Phase der wasserlöslichen Komponenten und eine Ölphase getrennt voneinander hergestellt werden. Im Anschluss werden die beiden Phasen miteinander gemischt unter Erhalt einer Wasser-in-Öl-Dispersion. Das Gemisch wird polymerisiert, indem ein Redoxinitiatorsystem zugegeben wird, gegebenenfalls kann anschließend noch ein thermischer Initiator zugegeben oder, fall bereits vorhanden, thermisch aktiviert werden.

In der wässrigen Phase sind vorzugsweise ein Kettentransfermittel, ein Vernetzer, ein kationisches Monomer und gegebenenfalls ein neutrales Monomer sowie das Assoziativmonomer enthalten sowie gegebenenfalls weitere Komponenten. Geeignete weitere Komponenten sind beispielsweise Komplexbildner für Salze wie Pentanatriumdiethylentriamin-pentaessigsäure oder Verbindungen, die zur Einstellung des pH-Wertes verwendet werden können, wie Zitronensäure. Weiterhin kann in der wässrigen Phase gegebenenfalls ein anionisches Monomer enthalten sein.

In der Ölphase sind vorzugsweise ein Emulgator, ein Stabilisator, ein hochsiedendes Öl, ein niedrigsiedendes Öl und/oder gegebenenfalls das Assoziativmonomer enthalten. Weiterhin kann in der Ölphase gegebenenfalls ein nicht-ionisches Monomer enthalten sein.

Emulgatoren, Stabilisatoren, niedrigsiedende Öle und hochsiedende Öle als solche sind dem Fachmann bekannt. Diese Verbindungen können einzeln oder in Form von Gemischen eingesetzt werden.

Typische Emulgatoren sind anionische Emulgatoren wie z. B. Natriumlaurylsulfat, Natriumtridecylethersulfate, Dioctylsulfosuccinat Natriumsalz und Natriumsalze von Alkylarylpolyethersulfonaten; und nichtionische Emulgatoren wie z. B. Alkylarylpolyetheralkohole und Ethylenoxid-Propylenoxid-Copolymere. Sorbitantrioleat ist ebenfalls als Emulgator geeignet.

Bevorzugte Emulgatoren weisen die folgende allgemeine Formel auf:

R-O-(CH₂-CHR'-O)ₙ-X,

worin R für C₆-C₃₀-Alkyl steht,
R' für Wasserstoff oder Methyl steht,
X für Wasserstoff oder SO₃M steht,
M für Wasserstoff oder ein Alkalimetall steht, und
n für eine ganze Zahl von 2 bis 100 steht.

Geeignete Stabilisatoren sind beispielsweise in EP-A 172 025 oder EP-A 172 724 beschrieben. Bevorzugte Stabilisatoren sind Copolymere aus Stearylmethacrylat und Methacrylsäure.

Als hochsiedende Öle eignen sich beispielsweise 2-Ethylhexylstearat sowie hydroerhitztes schweres Naphtha und als niedrigsiedende Öle beispielsweise dearomatisierte aliphatische Kohlenwasserstoffe oder Mineralöle von niedriger Viskosität.

In einer bevorzugten Ausführungsform der vorliegenden Erfindung wird bei der inversen Emulsionspolymerisation die Komponente b) (mindestens ein ethylenisch ungesättigtes Assoziativmonomer) auch oder ausschließlich in die Öl-Phase zugegeben.

Weiterhin ist es bevorzugt, dass nach der inversen Emulsionspolymerisation und vor der Aktivatorzugabe zumindest eine Teilmenge an Wasser und zumindest eine Teilmenge der niedrigsiedenden Bestandteile der Ölphase abdestilliert werden, insbesondere mittels LDP-Technologie (Liquid Dispersion Polymer Technologie). Die LDP-Technologie als solche ist dem Fachmann bekannt, sie ist beispielsweise in WO 2005/097834 beschrieben.

Ein weiterer Gegenstand der vorliegenden Erfindung ist das Verfahren als solches zur Herstellung der erfindungsgemäßen Verdicker gemäß den vorstehenden Ausführungen.

Ein weiterer Gegenstand der vorliegenden Erfindung sind tensidhaltige saure Formulierungen enthaltend mindestens einen erfindungsgemäßen Verdicker gemäß der vorstehenden Definitionen. Der pH-Wert der Formulierung beträgt 1 bis < 7.

Ein weiterer Gegenstand der vorliegenden Erfindung sind tensidhaltige alkalische Formulierungen enthaltend mindestens einen erfindungsgemäßen Verdicker gemäß den vorstehenden Definitionen. Der pH-Wert der Formulierung beträgt 7 bis 13.

In den erfindungsgemäßen tensidhaltigen sauren oder alkalischen Formulierungen können weitere Inhaltsstoffe enthalten sein, die dem Fachmann bekannt sind. Geeignete Inhaltsstoffe umfassen einen oder mehrere Stoffe aus der Gruppe der Gerüststoffe, Bleichmittel, Bleichaktivatoren, Enzyme, Elektrolyte, nichtwässrigen Lösungsmittel, pH-Stellmittel, Duftstoffe, Parfümträger, Fluoreszenzmittel, Farbstoffe, Hydrotrope, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Korrosionsinhibitoren, Antistatika, Bügelhilfsmittel, Phobier- und Imprägniermittel, Quell- und Schiebefestmittel sowie UV-Absorber.

In den erfindungsgemäßen tensidhaltigen Formulierungen, insbesondere in tensidhaltigen sauren Formulierungen, sind in einer Ausführungsform weniger als 1 Gew.-% Verdicker (bezogen auf die Gesamt-Formulierung) enthalten, vorzugsweise 0,1 bis < 1 Gew.-% an Verdicker.

Ein weiterer Gegenstand der vorliegenden Erfindung ist die Verwendung einer erfindungsgemäßen tensidhaltigen sauren Formulierung in der Haarkosmetik, beim Haarstyling, als Shampoo, als Weichspüler, als Conditioner, als Hautcreme, als Dusch-Gel, als Weichspüler für Wäsche, oder als saurer Reiniger, vorzugsweise für die Toilette oder das Bad.

Ein weiterer Gegenstand der vorliegenden Erfindung ist die Verwendung einer tensidhaltigen alkalischen Formulierung als Flüssigwaschmittel oder als Geschirrspülmittel für Maschinen - oder Handwäsche.

Ein weiterer Gegenstand der vorliegenden Erfindung ist die Verwendung des erfindungsgemäßen Verdickers als Viskositätsveränderer, zum Optimieren der Scherverdünnung, als Verdickungsmittel, zur Stabilisierung schwebender Inhaltsstoffe mit einer Größe im Bereich von Nanometer bis Millimeter und/oder in tensidhaltigen sauren oder alkalischen Formulierungen.

In der Beschreibung inklusive der Beispiele werden folgende Abkürzungen verwendet:

| | |
|---|---|
| **Monomere** | |
| ACM | Acrylamid |
| AA | Acrylsäure |
| MAA | Methacrylsäure |
| NaAc | Natriumacrylat |
| TMAEC | 2-Trimethylammoniumethylacrylatchlorid |
| TMAEMC | 2-Trimethylammoniumethylmethacrylatchlorid |
| BEM | Behenyl-25-Methacrylat |
| MBA | Methylen-bis-acrylamid (Vernetzer) |
| TAAC | Tetraallyl-ammoniumchlorid (Vernetzer) |
| NaHP | Natriumhypophosphit (Kettentransfermittel) |
| C16EO25MA | C₁₆-C₁₈-Fettalkohol-(Ethylenglykol)**₂₅** ethermethacrylat |
| **Sonstige** | |
| pphm | Teile pro hundert Teile Monomere (bzgl. Komponenten a) und b)) |
| VE | vollentsalzt |

Die Erfindung wird nachfolgend anhand der Beispiele verdeutlicht.

### Beispiele

### Vergleichsbeispiel V1

Synthese eines Verdickers/Polymers ausgehend von kationischen Monomeren ohne Assoziativmonomer, aber mit Vernetzer und Kettentransfermittel sowie steigender Polymerisationstemperatur.

Eine wässrige Phase wasserlöslicher Komponenten wird durch Beimischen folgender Komponenten hergestellt:
1,23 g (0,5 pphm) Zitronensäure-1-hydrat,
43,73 g (17,85 pphm) Wasser,
0,7 g (0,29 pphm) Pentanatrium-diethylentriamin-pentaessigsäure,
14,78 g (0,06 pphm) Methylenbisacrylamid (1 % in Wasser),
4,9 g (0,02 pphm) Tetra-allylammoniumchlorid (1 % in Wasser),
8 g (0,16 pphm) Natriumhypophosphit (5 % in Wasser), und
326,66 g (100 pphm) 2-Trimethylammoniumethyl methacrylat-chlorid (quarternisiertes Dimethylaminoethylmethacrylat) (TMAEMC, 75 % in Wasser).

Eine Ölphase wird durch Beimischen folgender Komponenten hergestellt:
8 g (2,45 pphm) Sorbitantrioleat (75 % in dearomatisiertem aliphatischem Kohlenwasserstoff [Exxsol D40]),
67,83 g (5,23 pphm) eines polymeren Stabilisators: Stearylmethacrylatmethacrylsäure-Copolymer (19% in dearomatisiertem aliphatischem Kohlenwasserstoff [Exxsol D40]),
151,29 g (61,75 pphm) 2-Ethylhexylstearat (Crodamol OS) und
60,17 g (24,56 pphm) dearomatisierter aliphatischer Kohlenwasserstoff [Exxsol D40].

Die zwei Phasen werden in einem Verhältnis aus 58,2 Teilen wässriger Phase zu 41,8 Teilen Ölphase unter hoher Scherung vermischt und somit eine Wasser-in-Öl-Emulsion hergestellt. Die entstehende Wasser-in-Öl-Emulsion wird in einen Reaktor gegeben, der mit Stickstoffsprühleitung, Rührer und Thermometer ausgestattet ist. Die Emulsion wird mit Stickstoff gespült, wodurch der Sauerstoff entfernt wird, und wird dann auf 20 °C abgekühlt.

Die Polymerisation wird durch Hinzufügen eines Redoxpaars aus
10 g (0,04 pphm) Natriummetabisulfit (1 % in dearomatisiertem aliphatischem Kohlenwasserstoff [Exxsol D40]) und
10 g (0,04 pphm) tertiär-Butylhydroperoxid (1 % in dearomatisiertem aliphatischem Kohlenwasserstoff [Exxsol D40]) erzielt.

Das Redoxpaar wird schrittweise so zugegeben, dass eine Temperaturerhöhung von 2 °C/min erfolgt. Nachdem die Isotherme erreicht ist, wird in 2 Schritten (der 2. Schritt nach 45 min) ein freier Radikalinitiator hinzugefügt (2,2'-Azobis(2-methylbutyronitril), CAS: 13472-08-7) und die Emulsion 75 Minuten lang auf 85 °C gehalten.

Mittels Vakuumdestillation werden Wasser und niedrigsiedende Bestandteile der Ölphase (Exxsol D40) entfernt.

Zum vakuumdestillierten Produkt wird 2-Ethylhexylstearat (Crodamol OS) hinzugefügt, wodurch ein Feststoffgehalt von 53,5 % erzielt wird. Danach werden 7% (bezogen auf den Gesamtmasseanteil dieses Produkts) eines fetthaltigen Alkoholalkoxylats [Alkohol C6-C17(Sekundär) Poly(3-6)ethoxylat: 97 % Sekundäralkoholethoxylat + 3 % Poly(ethylenoxid)], genannt Tergitol™ 15-S-7 (CAS-Nr. 84133-50-6), zur Herstellung eines Verdickers (Dispersion) mit 50 % Polymerfeststoffanteil hinzugefügt. Das Verhältnis von Aktivator zu kationischem Polymer beträgt somit 14,0 : 100 [Gew.-% / Gew.-%].

### Vergleichsbeispiel V2

Synthese eines Verdickers/Polymers ausgehend von kationischen Monomeren ohne Assoziativmonomer und Kettentransfermittel, aber mit Vernetzer sowie steigender Polymerisationstemperatur.

Die Synthese wird wie in V1 durchgeführt, jedoch mit dem Unterschied, dass kein Natriumhypophosphit (5 % in Wasser) und kein Tetraallylammoniumchlorid (1% in Wasser) dazugegeben und dafür die Wassermenge um 12,9 g Wasser erhöht wird. Das Verhältnis von Aktivator zu kationischem Polymer beträgt 14,0 : 100 [Gew.-% / Gew.-%].

### Vergleichsbeispiel V3

Synthese eines Verdickers/Polymers ausgehend von kationischen Monomeren ohne Assoziativmonomer, Kettentransfermittel und Vernetzer bei konstanter Polymerisationstemperatur.

Eine wässrige Phase wasserlöslicher Komponenten wird durch Beimischen folgender Komponenten hergestellt:
1,88 g (0,5 pphm) Zitronensäure-1-hydrat,
109,85 g (29,32 pphm) Wasser,
1,07 g (0,29 pphm) Pentanatriumdiethylentriamin-pentaessigsäure,
500,00 g (100 pphm) 2-Trimethylammoniumethyl methacrylat-chlorid (quarternisiertes Dimethylaminoethylmethacrylat) (TMAEMC 75 % in Wasser).

Eine Ölphase wird durch Beimischen folgender Komponenten hergestellt:
12,24 g (2,45 pphm) Sorbitantrioleat (75 % in dearomatisiertem aliphatischem Kohlenwasserstoff [Exxsol D40]),
103,83 g (5,22 pphm) eines polymeren Stabilisators: Stearylmethacrylatmethacrylsäure-Copolymer(19 % in dearomatisiertem aliphatischem Kohlenwasserstoff [Exxsol D40]),
231,57 g (61,75 pphm) 2-Ethylhexylstearat (Crodamol OS), und
92,10 g (24,56 pphm) dearomatisierter aliphatischer Kohlenwasserstoff [Exxsol D40]

Die zwei Phasen werden in einem Verhältnis aus 58,2 Teilen wässriger Phase zu 41,8 Teilen Ölphase unter hoher Scherung zur Herstellung einer Wasser-in-Öl-Emulsion vermischt. Die entstehende Wasser-in-Öl-Emulsion wird in einen Reaktor gegeben, der mit Stickstoffsprühleitung, Rührer und Thermometer ausgestattet ist. Die Emulsion wird mit Stickstoff gespült, wodurch der Sauerstoff entfernt wird.

Die Polymerisation wird durch Hinzufügen eines Redoxpaares erzielt, bestehend aus
13 g (0,05 pphm) Natriummetabisulfit (1 % in VE-Wasser) und
13g (0,05 pphm) tertiärem Butylhydroperoxid (1 % in VE-Wasser).
Die Geschwindigkeit für das Hinzufügen des Redoxpaares ist 13 g in 2 Stunden, wobei die Temperatur konstant auf 50°C gehalten wird. Danach wird in 2 Schritten (der 2. Schritt nach 45 min) ein freier Radikalinitiator (2,2'-Azobis(2-methylbutyronitrile), CAS: 13472-08-7) hinzugefügt und die Emulsion 75 Minuten lang auf 85°C gehalten.

Mittels Vakuumdestillation werden Wasser und niedrigsiedende Bestandteile der Ölphase (Exxsol D40) entfernt.

Zum vakuumdestillierten Produkt wird 2-Ethylhexylstearat (Crodamol OS) hinzugefügt, um einen Feststoffgehalt von 53,5 % zu erzielen.
Danach werden 7 % (bezogen auf den Gesamtmasseanteil dieses Produkts) eines fetthaltigen Alkoholalkoxylats [Alkohol C6-C17(Sekundär) Poly(3-6)ethoxylate : 97 % Sekundäralkoholethoxylat + 3 % Polyethylene oxide)], genannt Tergitol™ 15-S-7 (CAS-Nr. 84133-50-6), zur Herstellung eines Verdickers (Dispersion) mit 50 % Polymerfeststoffanteil hinzugefügt. Das Verhältnis von Aktivator zu kationischem Polymer beträgt somit 14,0 ; 100 [Gew.-% / Gew.-%].

### Vergleichsbeispiele V4 - V5

Wie V1, aber Änderungen gemäß Tabelle 1:

**Tabelle 1**

| Beispiele | TMAE MC (pphm) | MBA | TAAC | NaHP | Bemerkung |
|---|---|---|---|---|---|
| V4 | 50 | 0,0025 | 0,0025 | 0,001 | 50 pphm Acrylamid |
| V5 | 100 | 0,18 | 0,06 | 0,02 | |

Das Verhältnis von Aktivator zu kationischem Polymer beträgt in den Vergleichsbeispielen V4 bis V5 jeweils 14,0 : 100 [Gew.-% / Gew.-%].

### Beispiel 1

### Verdicker / Polymere ausgehend von kationischen Monomeren mit Assoziativmonomer:

Die folgenden Beispiele gemäß Tabelle 2 werden wie das Vergleichsbeispiel V3 hergestellt unter Berücksichtigung der angegebenen Änderungen in der Monomerzusammensetzung und in der Temperaturführung. Das Assoziativmonomer C16EO25MAc wird in die Ölphase gegeben. Es wird das Handelsprodukt Plex 6954 O eingesetzt, das 60 Gew.-% Assoziativmonomer sowie als Lösungsmittel Wasser und MAA im Verhältnis von ca. 1 : 1 enthält. Die Gewichtsangaben in Tabelle 2 beziehen sich auf die Menge an Assoziativmonomer ohne Lösungsmittel. Das Verhältnis von Aktivator zu kationischem Polymer beträgt in allen Beispielen gemäß Tabelle 1 jeweils 14,0 : 100 [Gew.-% / Gew.-%], soweit nicht anders angegeben haben die jeweiligen Verdicker (Dispersion) 50% Polymerfeststoffanteil. Vgl. bedeutet Vergleichsbeispiel.

**Tabelle 2**

| **Beispiele** | **C16EO25 MAc (pphm)** | **TMAEMC (pphm)** | **MBA** | **TAAC** | **NaHP** | **Bemerkung** |
|---|---|---|---|---|---|---|
| 1.1 | 0,19 | 99,75 | - | - | - | |
| 1.2 (Vgl.) | 0,19 | 99,75 | - | - | - | Temperaturführung wie V1; 30 % Polymerfeststoffanteil; Menge an Aktivator entsprechend angepasst |
| 1.5 | 0,19 | 99,75 | 0,06 | 0,02 | 0,05 | |
| 1.9 | 0,76 | 99,00 | | | | |
| 1.10 | 0,38 | 49,5 | | | | 50 pphm Acrylamid, |

### Allgemeine Messmethoden:

Soweit nicht anders angegeben, werden in den nachfolgenden Beispielen die folgenden allgemeinen Messmethoden verwendet:

### Bestimmung der Viskosität

Unter Berücksichtigung der Vorschriften nach DIN 51550, DIN 53018, DIN 53019 werden mit dem Brookfield Viskosimeter Modell DV II, wenn in den nachfolgenden Tabellen nicht anders angegeben, bei der Drehzahl 10 Umdrehungen pro Minute mit der Spindel Nr.2 die angegeben Viskositäten in mPas gemessen.

### Bestimmung der Scherverdünnung

Gemessen wird im Rotationsrheometer ASC (automatic sample changer) von Fa. Antonpaar, mit der Zylinder-Geometrie CC27, dem Radius des Messkörpers von 13,33 mm und dem Radius des Messbechers von 14,46 mm. Die Messtemperatur beträgt 23°C. Die Proben werden in stationärer Scherung beginnend bei kleiner Scherung hin zu großer (0,01 s⁻¹ -1000 s¹) und wieder zurück (1000 s⁻¹ - 0,01 s⁻¹) gemessen.

### Beispiel 2

Verdicker / Polymere ausgehend von kationischen Monomeren mit Assoziativmonomer sowie Einfluss der Aktivatormenge auf die Verdickungsgeschwindigkeit in wässrigen Formulierungen:
Die in Tabelle 3 aufgeführten Beispiele 2.1 bis 2.5 werden entsprechend zu Beispiel 1.5 aus Tabelle 2 hergestellt, wobei die nach der Destillation zugegebene Menge an Aktivator entsprechend der in der Tabelle 3 angegebenen Aktivatorkonzentration (A %) im Verdicker variiert wird (alle Angaben in Gew.-% bezogen auf die Menge an kationischem Polymer im Verdicker). Alle so hergestellten Verdicker (Dispersion) haben 50 % Polymerfeststoffanteil. Die Verdicker werden anschließend unter Rühren dem Wasser zugegeben. Diese dabei erhaltenen wässrigen Formulierungen enthalten 1 Gew.-% Verdicker zu 99 Gew.-% Wasser, also 0,5 Gew.-% Polymer zu 99,5 Gew.-% Wasser Vgl. bedeutet Vergleichsbeispiel.

**Tabelle 3**

| | | **Verdickungsgeschwindigkeit (Brookfieldvisk. mPas*s) der wässrigen Formulierungen** | | | | | | | |
|---|---|---|---|---|---|---|---|---|---|
| **Beispiele** | **A%** | **1 min.** | **2 min.** | **3 min.** | **5 min.** | **10 min.** | **20 min.** | **40 min.** | **1Std.** |
| 2.1 | 2,0 | 20 | 20 | 24 | 28 | 36 | 248 | 4800 | 7370 |
| 2.2 | 6,0 | 20 | 24 | 28 | 128 | 3640 | 8300 | 9630 | 10600 |
| 2.3 | 14,0 | 72 | 740 | 2600 | 6200 | 9100 | 11120 | 12220 | 12440 |
| 2.4 | 20.0 | 9100 | 10000 | 11060 | 11880 | 12540 | 12540 | 12780 | 12780 |
| 2.5 | 34,0 | 13280 | 13200 | 13140 | 13060 | 12920 | 12900 | 12800 | 12800 |

Tabelle 3 zeigt, dass eine Erhöhung der Aktivatormenge auf Werte über 10 % im erfindungsgemäßen Verdicker deutlich schneller zu höheren Viskositätswerten führt.

### Beispiel 3

### Anwendung der Verdicker / Polymere in Standard-Formulierungen von Weichspülern

### W1: Präparation eines di(hydrierten Talg)dimethylammoniumchlorid- (DHTDMAC) Weichspüler (4% Wirkanteil)

Zu 1890g deionisiertem, vorgewärmten Wasser werden 111g geschmolzenes 50 °C DHTDMAC (Arquad® 2HT-75) langsam unter Rühren hinzugegeben. Die Dispersion wird gerührt und 15 Minuten lang auf 50 °C unter stetigem Rühren erhitzt. Das Gemisch wird auf 30 °C unter Rühren abgekühlt. Der pH-Wert wird durch Zugabe von Zitronensäurelösung auf 4,0 eingestellt. Der Weichspüler wird durch Rühren homogenisiert.
LV Brookfield Viscosity (22 °C,, *30 Upm*) = 90 mPa·s.

### W3: Präparation eines Methyltris(hydroxyethyl)ammonium-di-Talg- Fettsäureestermethosulfats, teilweise hydrierter - Weichspüler (5,5% Wirkanteil)

Der Weichspüler hat einen pH von 2,7 und enthält 5,5 Gew.-% Methyltris(hydroxyethyl)ammonium-di-Talg- Fettsäureester-methosulfats (teilweise hydriert) und 94, 5 Gew.-% VE-Wasser.

### Zugabe des Verdickers zu den Weichspüler-Formulierungen W1 bis W3:

Die Verdicker gemäß Beispiel 1 (Tabelle 2) bzw. den Vergleichsbeispielen werden bei Raumtemperatur langsam zu der jeweiligen Weichspülerformulierung hinzugefügt und so lange gerührt, bis die Formulierung homogenisiert ist.
Die Brookfield-Viskosität wird einen Tag nach der Präparation gemessen. Die Ergebnisse sind in Tabelle 4 zusammengefasst.

**Tabelle 4: Verdickerleistung und Scherverdünnung in Weichspülern**

| Rheologie von Weichspülern enthaltend Verdicker / Polymere ausgehend von kationischen Monomeren: | | | | | | | |
|---|---|---|---|---|---|---|---|
| **Beispiel-Nr.** | **Formulierung** | **Verdicker-Nr.** | **Verdicker-Konzentration (%)** | **Vlskosltät bei 0,1 s⁻¹ (mPa*s)** | **Viskosität bei 10 s⁻¹ (mPa*s)** | **Viskosität bei 100 s⁻¹ (mPa*s)** | **Viskosität bei 1000 s⁻¹ (mPa*s)** |
| 3.1 (Vgl) | W3 | V1 | 0,5 | 27992 | 833 | 221 | 63 |
| 3.2 | W3 | 1.5 | 0,5 | 65986 | 1599 | 377 | 67 |
| 3.4 (Vgl) | W3 | V3 | 1,0 | 11062 | 901 | 213 | 56 |

Zusätzlich zur hohen Verdickungsleistung mit Assoziativmonomer und konstanter Temperaturfahrweise ist auch die relative prozentuale Scherverdünnung in den erfindungsgemäßen Verdickern viel ausgeprägter als in den Vergleichsbeispielen

### Beispiel 4

Anwendung der Verdicker / Polymere in Standard-Formulierungen von sauren Reinigern

### R1: Präparation eines sauren Reinigers gemäß der folgenden Zusammensetzung:

**pH = 5,3;**

| | |
|---|---|
| 12g | C₁₃-C₁₅-Oxoalkoholethoxylat mit 8 EO |
| 4g | C₁₃-C₁₅-Oxoalkoholethoxylat mit 5 EO |
| 2,5g | Ethylhexanolethoxylat |
| 81,5g | VE-Wasser |

### R2: Präparation eines sauren Reinigers gemäß der folgenden Zusammensetzung:

**pH = 1,8;**

| | |
|---|---|
| 10,3g | C₁₃-C₁₅-Oxoalkoholethoxylat mit 8 EO |
| 3,4g | C₁₃-C₁₅-Oxoalkoholethoxylat mit 5 EO |
| 2,2g | Ethylhexanolalkoxylat |
| 8,6g | Zitronensäure |
| 75,5g | VE-Wasser |

Die Zugabe der jeweiligen Verdicker zu diesen Standard-Formulierungen erfolgt wie vorstehend im Beispiel 3 beschrieben. Die Brookfield Viscosity wird einen Tag nach der Präparation gemessen. Die Ergebnisse sind in Tabelle 5 zusammengefasst.

**Tabelle 5: Verdickerleistung in sauren Reinigern**

| Rheologie von sauren Reinigern enthaltend Verdicker / Polymere ausgehend von kationischen Monomeren: | | | | | | |
|---|---|---|---|---|---|---|
| **BeisplelNr.** | **Formulierung** | **Verdicker-Nr.** | **VerdickerKonzentration (%)** | **Viskosität Bei 0,1 s⁻¹ (mPa*s)** | **Viskosität Bei 10 s⁻¹ (mPa*s)** | **Viskosität Bei 100 s⁻¹ (mPa*s)** |
| 4.1 (Vgl) | R1 | V1 | 1 | 16408 | 1850 | 618 |
| 4.2 | R1 | 1.5 | 1 | 140080 | 6100 | 1425 |
| 4.3 (Vgl) | R1 | V3 | 1 | 4620 | 810 | 232 |
| 4.5 (Vgl) | R2 | V1 | 1 | 7550 | 962 | 350 |
| 4.6 (Vgl) | R2 | V3 | 1 | 927 | 405 | 140 |
| 4.7 | R2 | 1.5 | 1 | 20918 | 1775 | 593 |

Zusätzlich zur hohen Verdickungsleistung ist auch die relative prozentuale Scherverdünnung in den erfindungsgemäßen Verdickern viel ausgeprägter als in den Vergleichsbeispielen .

### Beispiel 5

### Anwendung der Verdicker / Polymere in wässrigen Formulierungen

Die wässrigen Formulierungen werden hergestellt wie vorstehend unter Beispiel 2 beschrieben. Die Brookfield Viscosity wird einen Tag nach der Präparation gemessen. Die Ergebnisse sind in Tabelle 6 zusammengefasst.

**Tabelle 6**

| Rheologie von Verdickern / Polymere ausgehend von kationischen Monomeren in Wasser | | | | | | | |
|---|---|---|---|---|---|---|---|
| **Beispiel-Nr.** | **Formulierung** | **Verdicker-Nr.** | **VerdickerKonzentration (%)** | **Brookfield Spindel 3 (1 Upm) /mPas** | **Brookfield Spindel 3 (10 Upm) /mPas** | **Brookfield Spindel 3 (50 Upm) /mPas** | **Brookfield Spindel 3 (100 Upm) /mPas** |
| 5.1 (Vgl) | Wasser | V2 | 1 | 120 | 20 | 36 | 42 |
| 5.2 (Vgl) | Wasser | 1.2 | 1 | 480 | 300 | 182 | 145 |
| 5.3 | Wasser | 1.1 | 1 | 1320 | 596 | 274 | 190 |

Der Vergleich des Beispiels 5.3 mit dem Vergleichsbeispiel 5.2 zeigt, dass die Verwendung eines Polymers, das bei konstanter Temperatur polymerisiert wurde, zu einer ca. 30-%igen Verbesserung bei der Verdickerleistung führt.

### Beispiel 6

### Verdicker / Polymere ausgehend von kationischen Monomeren mit Assoziativmonomer sowie Einfluss der Aktivatormenge auf die Verdickungsgeschwindigkeit in Weichspüler-Formulierungen:

Die in Tabelle 3 beschriebenen Beispiele 2.1 bis 2.5 in wassriger Formulierung werden gemäß Tabelle 7 als Beispiele 6.1-6.5 in Weichspüler-Formulierungen mit Weichspülern (W3) gemäß Beispiel 3 analog durchgeführt: Wieder wird die Aktivatorkonzentration (A %) im Verdicker variiert, wobei die nach der Destillation zugegebene Menge an Aktivator entsprechend der in der Tabelle 7 angegebenen Aktivatorkonzentration (A %) im Verdicker variiert wird (alle Angaben in Gew.-% bezogen auf die Menge an kationischem Polymer im Verdicker). Alle so hergestellten Verdicker (Dispersion) haben 50% Polymerfeststoffanteil. Diese Verdicker werden unter Rühren dem Weichspüler W3 zugegeben. Die dabei erhaltenen verdickten Weichspüler-Formulierungen enthalten 1 Gew.-% Verdicker zu 99 Gew.-% Weichspüler W3, also 0,5 Gew.-% Polymer zu 99,5 Gew.-% Weichspüler W3.

**Tabelle 7**

| | | **Verdickungsgeschwindigkeit (Brookfieldvisk. mPas*s bei 10Upm)** | | | | | | | | |
|---|---|---|---|---|---|---|---|---|---|---|
| **Beispiel** | **A%** | **1 min.** | **2 min.** | **3 min.** | **5 min.** | **10 min.** | **20 min.** | **40 min.** | **1Std.** | **3Std.** |
| **6.1** | **3,0** | 152 | 244 | 368 | 644 | 1316 | 2376 | 3616 | 4930 | 9180 |
| **6.2** | **6,0** | 248 | 492 | 784 | 1400 | 2468 | 3336 | 4520 | 5020 | 7560 |
| **6.3** | **14,0** | 1900 | 2900 | 3680 | 4650 | 5200 | 5420 | 5600 | 5750 | 6280 |
| **6.4** | **20,0** | 2700 | 3572 | 3996 | 4600 | 4690 | 4650 | 4880 | 4820 | 5400 |
| **6.5** | **34,0** | 5600 | 5560 | 5480 | 5340 | 5200 | 4810 | 4810 | 4810 | 5000 |

Tabelle 7 zeigt, dass eine Erhöhung der Aktivatormenge auf Werte über 10 % deutlich schneller, also innerhalb von 3 Minuten, zu höheren 3-stelligen Viskositätswerten führt

### Beispiel 7

### Einfluss der Vernetzer-Menge auf die Löslichkeit der im Verdicker enthaltenen Polymere (ausgehend von kationischen Monomeren):

Die in Tabelle 10 aufgeführte Messung der löslichen Polymeranteile erfolgt gemäß der Methode von P. Schuck, ('Size-distribution analysis of macromolecules by sedimentation velocity ultracentrifugation and Lamm equation modeling', Biophysical Journal 78,(3) (2000), 1606-1619.).

**Tabelle 8: Bestimmung der Löslichkeit der im Verdicker enthaltenen TMAEMC-Copolymere mittels der analytischen Ultrazentrifuge (AUZ)**

| Beispiele | Polymer | lösliche TMAEMC-Copolymer in Verdicker (Dispersion) (% bezogen auf gesamtes Polymer) |
|---|---|---|
| 8.1 (Vgl.) | V1 | 24 |
| 8.2 (Vgl.) | V5 | <1 |
| 8.4 | 1.9 | 100 |
| 8.5 (vgl.) | V4 | 22 |
| 8.6 | 1.10 | 83 |

Die TMAEMC-Copolymere, die mit 800 pphm und mehr Vernetzer hergestellt wurden, enthalten zu weniger als 24 % lösliche Anteile. Die TMAEMC-Copolymere, die ohne Vernetzer und gegebenenfalls mit weniger als 1 pphm Associativmonomere hergestellt wurden, enthalten zu über 99 % lösliche Anteile. Acrylamid als Comonomer im TMAEMC-Copolymere reduziert die Löslichkeit des Copolymeren.

## Patentansprüche

1. Verdicker herstellbar nach einem Verfahren, **dadurch gekennzeichnet, dass** ein kationisches Polymer durch eine inverse Emulsionspolymerisation von
a) mindestens einem wasserlöslichen ethylenisch ungesättigten Monomeren umfassend mindestens ein kationisches Monomer, gegebenenfalls mindestens ein anionisches Monomer und/oder gegebenenfalls mindestens ein nicht-ionisches Monomer,
b) mindestens einem ethylenisch ungesättigten Assoziativmonomeren,
c) gegebenenfalls mindestens einem Vernetzer,
d) gegebenenfalls mindestens einem Kettentransfermittel,
erhalten wird, wobei die Temperatur während der inversen Emulsionspolymerisation konstant gehalten wird und mindestens 40 °C, vorzugsweise 50 bis 90 °C, beträgt und nach Beendigung der inversen Emulsionspolymerisation die Aktivatorzugabe unter Erhalt des Verdickers erfolgt, und
wobei der Verdicker mindestens einen Aktivator enthält und der Aktivator ein Tensid ist.

2. Verdicker gemäß Anspruch 1, **dadurch gekennzeichnet, dass** nach der inversen Emulsionspolymerisation und vor der Aktivatorzugabe, zumindest eine Teilmenge an Wasser und zumindest eine Teilmenge der niedrigsiedenden Bestandteile der Ölphase abdestilliert werden.

3. Verdicker gemäß Anspruch 1 oder 2, **dadurch gekennzeichnet, dass** das Verfahren auf der LDP-Technologie (liquid dispersion polymer Technologie) beruht.

4. Verdicker gemäß einem der Ansprüche 1 bis 3, **dadurch gekennzeichnet, dass** bei der inversen Emulsionspolymerisation die Komponente b) in die Öl-Phase zugegeben wird.

5. Verdicker gemäß einem der Ansprüche 1 bis 4, **dadurch gekennzeichnet, dass** der Aktivator ausgewählt ist aus Fettalkoholalkoxylaten, Alkylglykosiden, Alkylcarboxylaten, Alkylbenzolsulfonaten, sekundären Alkansulfonaten und Fettalkoholsulfaten, vorzugsweise ausgewählt aus Fettalkoholalkoxylaten.

6. Verdicker gemäß einem der Ansprüche 1 bis 5, **dadurch gekennzeichnet, dass** ein Gemisch von mindestens 2 Aktivatoren eingesetzt wird, wobei mindestens ein Aktivator einen HLB-Wert (hydrophil-lipophil-balance-Wert) von > 12 bis 20 und mindestens ein Aktivator einen HLB-Wert von 1 bis 12 aufweist.

7. Verdicker gemäß einem der Ansprüche 1 bis 6, **dadurch gekennzeichnet, dass** das kationische Polymer in der Ölphase dispergiert vorliegt, vorzugsweise als inverse Dispersion, Wasser-in-Öl-Dispersion oder als dispergiertes wasserfreies kationisches Polymer in Öl.

8. Verdicker gemäß einem der Ansprüche 1 bis 7, **dadurch gekennzeichnet, dass** im kationischen Polymer das ethylenisch ungesättigte Assoziativmonomer gemäß Komponente b) ausgewählt ist aus einer Verbindung gemäß Formel (I)
R-O-(CH₂-CHR'-O)ₙ-CO-CR"=CH₂ (I)
wobei
R gleich C₆ - C₅₀ - Alkyl, vorzugsweise C₈ - C₃₀- Alkyl, insbesondere C₁₆ - C₂₂- Alkyl, ist,
R' gleich H oder C₁ - C₄ - Alkyl, vorzugsweise H, ist,
R" gleich H oder Methyl ist,
n eine ganze Zahl von 0 bis 100, vorzugsweise 3 bis 50, insbesondere 25, ist,

9. Verdicker gemäß einem der Ansprüche 1 bis 8, **dadurch gekennzeichnet, dass** die in Wasser löslichen Anteile des kationischen Polymers mehr als 25 Gew.-% ( bezogen auf das Gesamtgewicht des kationischen Polymers) betragen.

10. Verdicker gemäß einem der Ansprüche 1 bis 9, **dadurch gekennzeichnet, dass** im kationischen Polymer das kationische Monomer gemäß Komponente a) ausgewählt ist aus einer Verbindung gemäß Formel (II) 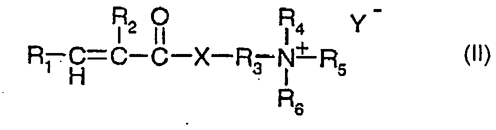 wobei
R₁ gleich H oder C₁ - C₄ - Alkyl ist,
R₂ gleich H oder Methyl ist,
R₃ gleich C₁ - C₄ - Alkylen ist,
R₄, R₅ und R₆ unabhängig voneinander H oder C₁ - C₃₀ - Alkyl sind,
X gleich -O- oder -NH- ist und
Y gleich Cl; Br; I; Hydrogensulphat oder Methosulfat ist.

11. Verdicker gemäß Anspruch 10, **dadurch gekennzeichnet, dass** im kationischen Monomer gemäß Formel (II)
i) R₁ und R₂ gleich H oder
ii) R₁ gleich H und R₂ gleich CH₃ sind.

12. Verdicker gemäß einem der Ansprüche 1 bis 11, **dadurch gekennzeichnet, dass** im kationischen Polymer die Komponente a) mindestens ein nicht-ionisches Monomer umfasst, wobei das nicht-ionische Monomer ausgewählt ist aus N-Vinylpyrrolidon, N-Vinylimidazol oder einer Verbindung gemäß der Formel (III) 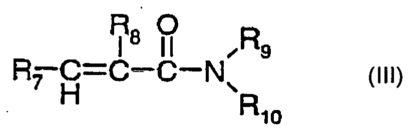 wobei
R₇ gleich H oder C₁ - C₄ - Alkyl ist,
R₈ gleich H oder Methyl ist, und
R₉ und R₁₀ unabhängig voneinander H oder C₁ - C₃₀ - Alkyl sind.

13. Verdicker gemäß einem der Ansprüche 1 bis 12, **dadurch gekennzeichnet, dass** im kationischen Polymer die Komponente a) 30 bis 99,5 Gew.-% von mindestens einem kationischen Monomeren und 0,5 bis 70 Gew.-% von mindestens einem nicht-ironischen Monomeren umfasst.

14. Verdicker gemäß einem der Ansprüche 1 bis 13, **dadurch gekennzeichnet, dass** im kationischen Polymer der Vernetzer (Komponente c) ausgewählt ist aus
Divinylbenzol; Tetraallyl-Ammoniumchlorid; Allylacrylaten; Allylmethacrylaten; Diacrylaten und Dimethacrylate von Glykolen oder Polyglykolen; Butadien; 1,7-Octadien, Allyl-Acrylamiden oder Allyl-Methacrylamiden; Bisacrylamidoessigsäure; N,N'-Methylen-bisacrylamid oder Polyolpolyallylethern wie Polyallylsaccharose oder Pentaerythritoltriallylether.

15. Verdicker gemäß einem der Ansprüche 1 bis 14, **dadurch gekennzeichnet, dass** im kationischen Polymer das Kettentransfermittel (Komponente d) ausgewählt ist aus Mercaptanen, Milchsäure, Ameisensäure, Isopropanol oder Hypophosphiten.

16. Verdicker gemäß einem der Ansprüche 1 bis 15, **dadurch gekennzeichnet, dass** das Verhältnis von Aktivator zu kationischem Polymer > 10 zu 100 [Gew.-% / Gew.-%] beträgt.

17. Tensidhaltige saure Formulierung enthaltend mindestens einen Verdicker gemäß einem der Anspruch 1 bis 16, wobei der pH-Wert der Formulierung 1 bis <7 beträgt.

18. Verwendung einer tensidhaltigen sauren Formulierung gemäß Anspruch 17 in der Haarkosmetik, beim Haarstyling, als Shampoo, als Weichspüler, als Conditioner, als Hautcreme, als Dusch-Gel, als Weichspüler für Wäsche, oder als saurer Reiniger, vorzugsweise für die Toilette oder das Bad.

19. Tensidhaltige alkalische Formulierung enthaltend mindestens einen Verdicker gemäß einem der Anspruch 1 bis 16, wobei der pH-Wert der Formulierung 7 bis 13 beträgt.

20. Verwendung einer tensidhaltigen alkalischen Formulierung gemäß Anspruch 19 als Flüssigwaschmittel oder als Geschirrspülmittel für Maschinen - oder Handwäsche.

21. Verwendung eines Verdickers gemäß einem der Ansprüche 1 bis 16 als Viskositätsveränderer, zum Optimieren der Scherverdünnung, als Verdickungsmittel, zur Stabilisierung schwebender Inhaltsstoffe mit einer Größe im Bereich von Nanometer bis Millimeter und/oder in tensidhaltigen sauren oder alkalischen Formulierungen.

## Claims

1. A thickener preparable by a process which comprises obtaining a cationic polymer by inverse emulsion polymerization of
a) at least one water-soluble ethylenically unsaturated monomer comprising at least one cationic monomer, optionally at least one anionic monomer and/or optionally at least one nonionic monomer,
b) at least one ethylenically unsaturated associative monomer,
c) optionally at least one crosslinker,
d) optionally at least one chain transfer agent,
the temperature being kept constant during the inverse emulsion polymerization and being at least 40°C, preferably 50 to 90°C, and, after the inverse emulsion polymerization has ended, activator being added to obtain the thickener, and
wherein the thickener comprises at least one activator and the activator is a surfactant.

2. The thickener according to claim 1, wherein the inverse emulsion polymerization is followed and the activator addition is preceded by distillative removal of at least a portion of water and at least a portion of the low-boiling constituents of the oil phase.

3. The thickener according to claim 1 or 2, wherein the process is based on LDP (liquid dispersion polymer) technology.

4. The thickener according to any of claims 1 to 3, wherein component b) is added to the oil phase in the inverse emulsion polymerization.

5. The thickener according to any of claims 1 to 4, wherein the activator is selected from fatty alcohol alcoxylates, alkyl glycosides, alkyl carboxylates, alkylbenzenesulfonates, secondary alkanesulfonates and fatty alcohol sulfates, preferably selected from fatty alcohol alcoxylates.

6. The thickener according to any of claims 1 to 5, wherein a mixture of at least two activators is used, at least one activator having an HLB (hydrophilic-lipophilic balance) value of > 12 to 20 and at least one activator an HLB value of 1 to 12.

7. The thickener according to any of claims 1 to 6, wherein the cationic polymer is present dispersed in the oil phase, preferably as an inverse dispersion, water-in-oil dispersion, or as a dispersed anhydrous cationic polymer in oil.

8. The thickener according to any of claims 1 to 7, wherein the ethylenically unsaturated associative monomer according to component b) in the cationic polymer is selected from a compound of the formula (I)
R-O-(CH₂-CHR'-O)ₙ-CO-CR"=CH₂ (I)
where
R is C₆ - C₅₀ - alkyl, preferably C₈ - C₃₀-alkyl, especially C₁₆ - C₂₂- alkyl,
R' is H or C₁ - C₄ - alkyl, preferably H,
R'' is H or methyl,
n is an integer from 0 to 100, preferably 3 to 50, especially 25.

9. The thickener according to any of claims 1 to 8, wherein the water-soluble components of the cationic polymer are more than 25% by weight (based on the total weight of the cationic polymer).

10. The thickener according to any of claims 1 to 9, wherein the cationic monomer according to component a) in the cationic polymer is selected from a compound of the formula (II) 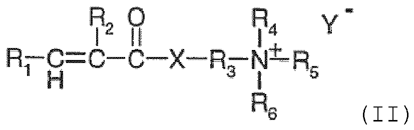 where
R₁ is H or C₁ - C₄ - alkyl,
R₂ is H or methyl,
R₃ is C₁ - C₄ - alkylene,
R₄, R₅ and R₆ are each independently H or C₁ - C₃₀-alkyl,
X is -O- or -NH- and
Y is Cl; Br; I; hydrogensulfate or methosulfate.

11. The thickener according to claim 10, wherein, in the cationic monomer of the formula (II),
i) R₁ and R₂ are each H or
ii) R₁ is H and R₂ is CH₃.

12. The thickener according to any of claims 1 to 11, wherein the component a) in the cationic polymer comprises at least one nonionic monomer, the nonionic monomer being selected from N-vinylpyrrolidone, N-vinylimidazole or a compound of the formula (III) 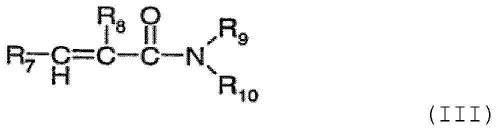 where
R₇ is H or C₁ - C₄ - alkyl,
R₈ is H or methyl, and
R₉ and R₁₀ are each independently H or C₁ - C₃₀-alkyl.

13. The thickener according to any of claims 1 to 12, wherein component a) in the cationic polymer comprises 30 to 99.5% by weight of at least one cationic monomer and 0.5 to 70% by weight of at least one nonionic monomer.

14. The thickener according to any of claims 1 to 13, wherein the crosslinker (component c)) in the cationic polymer is selected from
divinylbenzene; tetraallylammonium chloride; allyl acrylates; allyl methacrylates; diacrylates and dimethacrylates of glycols or polyglycols; butadiene; 1,7-octadiene, allylacrylamides or allylmethacrylamides; bisacrylamidoacetic acid; N,N'-methylenebisacrylamide, or polyol polyallyl ethers such as polyallyl sucrose or pentaerythritol triallyl ether.

15. The thickener according to any of claims 1 to 14, wherein the chain transfer agent (component d) in the cationic polymer is selected from mercaptans, lactic acid, formic acid, isopropanol or hypophosphites.

16. The thickener according to any of claims 1 to 15, wherein the ratio of activator to cationic polymer is > 10 to 100 [% by weight / % by weight].

17. A surfactant-containing acidic formulation comprising at least one thickener according to any of claims 1 to 16, the pH of the formulation being 1 to <7.

18. The use of a surfactant-containing acidic formulation according to claim 17 in hair cosmetics, in hair styling, as a shampoo, as a softener, as a conditioner, as a skin cream, as a shower gel, as a fabric softener for laundry, or as an acidic detergent, preferably for toilets or baths.

19. A surfactant-containing alkaline formulation comprising at least one thickener according to any of claims 1 to 16, the pH of the formulation being 7 to 13.

20. The use of a surfactant-containing alkaline formulation according to claim 19 as a liquid washing composition or as a machine or manual dishwashing detergent.

21. The use of a thickener according to any of claims 1 to 16 as a viscosity modifier, for optimization of shear dilution, as a thickening agent, for stabilization of suspended constituents having a size in the range from nanometers to millimeters and/or in surfactant-containing acidic or alkaline formulations.

## Revendications

1. Épaississant pouvant être fabriqué par un procédé, **caractérisé en ce qu'**un polymère cationique est obtenu par une polymérisation en émulsion inverse de
a) au moins un monomère éthyléniquement insaturé soluble dans l'eau comprenant au moins un monomère cationique, éventuellement au moins un monomère anionique et/ou éventuellement au moins un monomère non ionique,
b) au moins un monomère associatif éthyléniquement insaturé,
c) éventuellement au moins un agent de réticulation,
d) éventuellement au moins un agent de transfert de chaînes,
la température étant maintenue constante pendant la polymérisation en émulsion inverse et étant d'au moins 40 °C, de préférence de 50 à 90 °C, et l'ajout d'un activateur pour obtenir l'épaississant ayant lieu après la fin de la polymérisation en émulsion inverse, et l'épaississant contenant au moins un activateur et l'activateur étant un tensioactif.

2. Épaississant selon la revendication 1, **caractérisé en ce qu'**au moins une partie de l'eau et au moins une partie des constituants de faible point d'ébullition sont éliminées de la phase huileuse par distillation après la polymérisation en émulsion inverse et avant l'ajout de l'activateur.

3. Épaississant selon la revendication 1 ou 2, **caractérisé en ce que** le procédé est fondé sur la technologie LDP (technologie Liquid Dispersion Polymer).

4. Épaississant selon l'une quelconque des revendications 1 à 3, **caractérisé en ce que** le composant b) est ajouté dans la phase huileuse lors de la polymérisation en émulsion inverse.

5. Épaississant selon l'une quelconque des revendications 1 à 4, **caractérisé en ce que** l'activateur est choisi parmi les alcoxylates d'alcools gras, les glycosides d'alkyle, les carboxylates d'alkyle, les sulfonates d'alkylbenzène, les sulfonates d'alcanes secondaires et les sulfates d'alcools gras, de préférence parmi les alcoxylates d'alcools gras.

6. Épaississant selon l'une quelconque des revendications 1 à 5, **caractérisé en ce qu'**un mélange d'au moins 2 activateurs est utilisé, au moins un activateur présentant une valeur HLB (équilibre hydrophile-lipophile) de > 12 à 20, et au moins un activateur présentant une valeur HLB de 1 à 12.

7. Épaississant selon l'une quelconque des revendications 1 à 6, **caractérisé en ce que** le polymère cationique est présent sous forme dispersée dans la phase huileuse, de préférence sous la forme d'une dispersion inverse, d'une dispersion eau dans huile ou sous la forme d'un polymère cationique anhydre dispersé dans de l'huile.

8. Épaississant selon l'une quelconque des revendications 1 à 7, **caractérisé en ce que**, dans le polymère cationique, le monomère associatif éthyléniquement insaturé selon le composant b) est choisi parmi un composé de formule (I)
R-O-(CH₂-CHR'-O)ₙ-CO-CR"=CH₂ (I)
dans laquelle
R représente alkyle en C₆-C₅₀, de préférence alkyle en C₈-C₃₀, notamment alkyle en C₁₆-C₂₂,
R' représente H ou alkyle en C₁-C₄, de préférence H,
R" représente H ou méthyle,
n représente un nombre entier de 0 à 100, de préférence de 3 à 50, notamment 25.

9. Épaississant selon l'une quelconque des revendications 1 à 8, **caractérisé en ce que** les proportions solubles dans l'eau du polymère cationique sont supérieures à 25 % en poids (par rapport au poids total du polymère cationique).

10. Épaississant selon l'une quelconque des revendications 1 à 9, **caractérisé en ce que**, dans le polymère cationique, le monomère cationique selon le composant a) est choisi parmi un composé de formule (II) 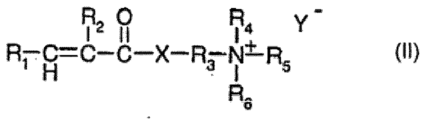 dans laquelle
R₁ représente H ou alkyle en C₁-C₄,
R₂ représente H ou méthyle,
R₃ représente alkylène en C₁-C₄,
R₄, R₅ et R₆ représentent indépendamment les uns des autres H ou alkyle en C₁-C₃₀,
X représente -O- ou -NH-, et
Y représente Cl, Br, I, hydrogénosulfate ou méthosulfate.

11. Épaississant selon la revendication 10, **caractérisé en ce que**, dans le monomère cationique de formule (II),
i) R₁ et R₂ représentent H, ou
ii) R₁ représente H et R₂ représente CH₃.

12. Épaississant selon l'une quelconque des revendications 1 à 11, **caractérisé en ce que**, dans le polymère cationique, le composant a) comprend au moins un monomère non ionique, le monomère non ionique étant choisi parmi la N-vinylpyrrolidone, le N-vinylimidazole ou un composé de formule (III) 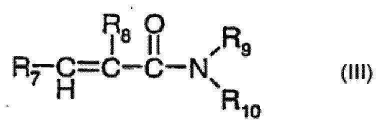 dans laquelle
R₇ représente H ou alkyle en C₁-C₄,
R₈ représente H ou méthyle, et
R₉ et R₁₀ représentent indépendamment l'un de l'autre H ou alkyle en C₁-C₃₀.

13. Épaississant selon l'une quelconque des revendications 1 à 12, **caractérisé en ce que**, dans le polymère cationique, le composant a) comprend 30 à 99,5 % en poids d'au moins un monomère cationique et 0,5 à 70 % en poids d'au moins un monomère non ionique.

14. Épaississant selon l'une quelconque des revendications 1 à 13, **caractérisé en ce que**, dans le polymère cationique, l'agent de réticulation (composant c) est choisi parmi
le divinylbenzène, le chlorure de tétrallyl-ammonium, les acrylates d'allyle, les méthacrylates d'allyle, les diacrylates et diméthacrylates de glycols ou de polyglycols, le butadiène, le 1,7-octadiène, les allyl-acrylamides ou les allyl-méthacrylamides, l'acide bisacrylamidoacétique, le N,N'-méthylène-bisacrylamide ou les éthers polyallyliques de polyols tels que le polyallylsaccharose ou l'éther triallylique de pentaérythritol.

15. Épaississant selon l'une quelconque des revendications 1 à 14, **caractérisé en ce que**, dans le polymère cationique, l'agent de transfert de chaînes (composant d) est choisi parmi les mercaptans, l'acide lactique, l'acide formique, l'isopropanol ou les hypophosphites.

16. Épaississant selon l'une quelconque des revendications 1 à 15, **caractérisé en ce que** le rapport entre l'activateur et le polymère cationique est de > 10 à 100 [% en poids/% en poids].

17. Formulation acide contenant des tensioactifs, contenant au moins un épaississant selon l'une quelconque des revendications 1 à 16, dans laquelle le pH de la formulation est de 1 à < 7.

18. Utilisation d'une formulation acide contenant des tensioactifs selon la revendication 17 en cosmétique capillaire, pour la coiffure, en tant que shampoing, en tant qu'adoucissant, en tant qu'après-shampoing, en tant que crème pour la peau, en tant que gel douche, en tant qu'adoucissant pour le linge ou en tant que nettoyant acide, de préférence pour la toilette ou le bain.

19. Formulation alcaline contenant des tensioactifs, contenant au moins un épaississant selon l'une quelconque des revendications 1 à 16, dans laquelle le pH de la formulation est de 7 à 13.

20. Utilisation d'une formulation alcaline contenant des tensioactifs selon la revendication 19 en tant que détergent liquide ou liquide vaisselle pour machines ou pour lavage à la main.

21. Utilisation d'un épaississant selon l'une quelconque des revendications 1 à 16 en tant que modificateur de viscosité, pour l'optimisation de la fluidification par cisaillement, en tant qu'agent épaississant, pour la stabilisation d'ingrédients flottants d'une taille dans la plage allant des nanomètres aux millimètres et/ou dans des formulations acides ou alcalines contenant des tensioactifs.
